# EUROPEAN PATENT APPLICATION

(11) **EP 4 293 115 A1**
(43) Date of publication of application: **20.12.2023**
(21) Application number: 22738917.8
(22) Date of filing: 06.01.2022
(51) Int. Cl.: C12N 15/113, C12N 15/77, C12N 1/21, C12P 13/04, C12P 7/44, C12R 1/15

(54) **POLYNUCLEOTIDE HAVING PROMOTER ACTIVITY AND USE THEREOF IN PRODUCTION OF TRAGET COMPOUNDS**

(30) Priority: 12.01.2021 CN 202110036121; 10.05.2021 CN 202110504946; 26.07.2021 CN 202110841507
(71) Applicant: Tianjin Institute of Industrial Biotechnology, Chinese Academy of Sciences, Tianjin Airport Economic Area Tianjin 300308 (CN)
(72) Inventor: ZHENG, Ping, Tianjin 300308 (CN); CHEN, Jiuzhou, Tianjin 300308 (CN); HUANG, Jingwen, Tianjin 300308 (CN); SUN, Jibin, Tianjin 300308 (CN); ZHOU, Wenjuan, Tianjin 300308 (CN); LIU, Jiao, Tianjin 300308 (CN); WANG, Yu, Tianjin 300308 (CN); SHI, Tuo, Tianjin 300308 (CN); MA, Yanhe, Tianjin 300308 (CN)
(74) Representative: Weickmann & Weickmann PartmbB
(86) International application number: PCT/CN2022/070499
(87) International publication number: WO 2022/152036

(57) **Abstract**

Provided are a polynucleotide having promoter activity and use thereof in the production of target compounds. Specifically, provided are a polynucleotide having promoter activity, a transcription expression cassette, a recombinant expression vector, and a recombinant host cell comprising the polynucleotide having promoter activity, a method for regulating the transcription of a target gene, a method for preparing a protein, and a method for producing target compounds. The provided polynucleotide having promoter activity is a high salt and high osmolality-inducible promoter, and has an enhanced promoter activity in an environment of increased salt concentration and osmolality. The polynucleotide is operably ligated to the target gene, can significantly improve the expression intensity of the target gene in the stress environment of high salt and high osmolality, thereby stably and efficiently producing a downstream product, and effectively solving the current problems of adding expensive inducers such as IPTG and causing toxicity to a strain.

## Description

### CROSS-REFERENCE TO RELATED APPLICATION

The present disclosure is based upon and claims the benefits of the priorities of Chinese Patent Application No. 202110036121.1, titled "POLYNUCLEOTIDE HAVING PROMOTER ACTIVITY AND USE THEREOF IN PRODUCTION OF TARGET COMPOUNDS", filed on January 12, 2021; Chinese Patent Application No. 202110504946.1, titled "POLYNUCLEOTIDE HAVING PROMOTER ACTIVITY AND USE THEREOF IN PRODUCTION OF TARGET COMPOUNDS", filed on May 10, 2021; and Chinese Patent Application No. 202110841507.X, titled "MUTATED HYPERTONIC INDUCIBLE PROMOTER PproP AND APPLICATION THEREOF", filed on July 26, 2021; the entire contents of which are incorporated herein by reference.

### TECHNICAL FIELD

The present disclosure relates to the field of biotechnology and genetic engineering technology, in particular to a polynucleotide having promoter activity, a transcription expression cassette, a recombinant expression vector, a recombinant host cell comprising the polynucleotide having promoter activity, a method for regulating the transcription of a target gene, a method for preparing a protein, and a method for producing target compounds.

### BACKGROUND

*Corynebacterium,* in particular the nonpathogenic *Corynebacterium glutamicum,* is recognized by the US FDA to be safe for food and is currently one of the most commonly used strains in the fermentation industry due to its strong capability of amino acid synthesis, and is widely used in the industrial production of chemicals including proteins, amino acids, organic acids, etc. The regulation and optimization of target gene expression are crucial for increasing protein or product synthesis; and the promoter element is an important tool for regulating gene expression. By far, a series of strong promoters^{[1-2]} or constitutive promoters^{[3]} have been identified or developed in *Corynebacterium glutamicum* to regulate the expression of key genes in metabolic pathways. Although these promoter elements are capable of fine regulation of the expression of target genes, the use of constitutive promoters is rather limited in the synthesis of some toxic proteins or metabolic products. Moreover, the expression of strong promoters or constitutive promoters often cause a great metabolic burden on engineered strains.

Inducible promoters are capable of controlling the initiation time of transcription, and are therefore more condusive to the regulation and redistribution of metabolic fluxes of strains. By far, inducible promoters such as *tac, trc* have been widely used in metabolic regulation of *Corynebacterium glutamicum.* However, such promoters usually require adding additional expensive inducers such as IPTG The addition of inducers may also cause some toxicity to the strains or lead to interference of the fermentation system. Therefore, it is critical for the construction of industrial strains to develop a self-induction system under the industrial fermentation conditions. At the current stage, self-inducible promoters in a part of *Corynebacterium glutamicum* have been reported in researches, such as lysine-inducible promoters^{[4]}, growth-regulated promoters^{[5-6]}, and the like. But these self-inducible promoters are rather scarce in number and respond to relatively a narrow range of conditions, and therefore are not universally applicable in more fermentation systems and product synthesis.

Under the conditions of strictly controlled pH and dissolved oxygen in the fermenter, the yield of glutamic acid, lysine, or other commodity chemicals from *Corynebacterium glutamicum* can reach 100 g/L or 200 g/L or more^{[7-8]}. Accordingly, the stress of high salt and high osmolality will certainly arise at a late stage of fermentation due to accumulation of high-density products and intermediate metabolite and continuous feeding of the substrate. In addition, the high salt and high osmolality condition is also an environmental induction factor confronted by almost all industrial strains at the late stage of fermentation^{[9]}. By far, there is no prior application of a high salt and high osmolality-inducible promoter of *Corynebacterium glutamicum* in the regulation of the expression of a target gene.

Therefore, identifying a high salt and high osmolality-inducible promoter and developing and constructing a self-induction system for the high salt and high osmolality condition at a late stage of fermentation not only makes it possible to add an applicable self-induction system, but provides a self-inducible element universally adaptive for the development of all industrial strains. This is also a key problem urgent to be solved in the current development of industrial stains of *Corynebacterium glutamicum.*

### Reference Documents

[1] CN101605890A.
[2] Becker, J., et al., Metab. Eng., 2011, 13, 159-168.
[3] Rytter, J. V, et al., Appl. Microbiol. Biotechnol., 2014, 98, 2617-2623.
[4] CN101087881A.
[5] Kim, M. J., et al., Appl. Microbiol. Biotechnol., 2016, 100, 4473-4483.
[6] Ma, Y. C., et al., Microb. Cell Fact., 2018, 17.
[7] Xu, J. Z., et al., Microb Cell Fact, 2020, 19, 39.
[8] Hu, T.H., etal., China Brewing, 2018, 37(10), 51-56.
[9] Varela C et al. Applied Microbiology And Biotechnology 2003, 60(5): 547-555.

### SUMMARY

### Technical problem

In view of the technical problem existing in the prior art, for example, the inducible promoters such as *tac*, *trc* used in industrial fermentation requiring the addition of IPTG or other expensive inducers and the addition of the inducers causing toxicity to the strains, the present disclosure provides a polynucleotide having promoter activity which exhibits an enhanced promoter activity in an environment of increased salt concentration and osmolality. By operably ligating the polynucleotide to protein-coding genes involved in amino acid synthesis, it can realize efficient expression of the protein-coding genes in high salt and high osmolality environments, thereby effectively solving the problem that the existing inducible promoters require adding expensive inducers, and the addition of inducers causing toxicity to the strain.

In some embodiments, the polynucleotide having promoter activity provided by the present disclosure is a mutant obtained based on the polynucleotide having the sequence as shown in any one of SEQ ID NOs: 1 to 3. The mutant according to the present disclosure exhibits an enhanced promoter activity in environments of increased salt concentration and osmolality. By operatively ligating the mutant to a target gene, it can express the target gene in high salt and high osmolality environments and save the use of expensive inducers such as IPTG, and provide a novel high salt or high osmolality-inducible promoter for efficient production of target compounds. Further, the mutant has significantly increased promoter activity as compared to the wild-type promoter, and therefore is of significant value in industrial application.

### Solution to the problem

(1) A polynucleotide having promoter activity, wherein the polynucleotide is any one selected from a group consisting of (i) to (iv):
   (i) comprising a nucleotide sequence as shown in any one sequence of SEQ ID NOs: 1 to 3;
   (ii) comprising a reverse complementary sequence to the nucleotide sequence as shown in any one sequence of SEQ ID NOs: 1 to 3;
   (iii) a reverse complementary sequence of a sequence capable of hybridizing with the nucleotide sequence as shown in (i) or (ii) under high-stringent hybridization conditions or very high-stringent hybridization conditions;
   (iv) a sequence having at least 80%, optionally at least 90%, preferably at least 95%, more preferably at least 97%, further preferably at least 98%, most preferably at least 99% sequence identity to the nucleotide sequence as set forth in (i) or (ii),
(2) The polynucleotide having promoter activity according to (1), wherein the polynucleotide has an enhanced promoter activity in an environment of increased salt concentration or osmolality.
(3) A transcription expression cassette, wherein the transcription expression cassette comprises the polynucleotide having promoter activity according to (1) or (2), optionally, the transcription expression cassette further comprises a protein-coding gene, the protein-coding gene is operably ligated with the polynucleotide having promoter activity.
(4) A recombinant expression vector, wherein the recombinant expression vector comprises the polynucleotide having promoter activity according to (1) or (2), or the transcription expression cassette according to (3).
(5) A recombinant host cell, wherein the recombinant host cell comprises the transcription expression cassette according to (3), or the recombinant expression vector according to (4).
(6) The recombinant host cell according to (5), wherein the host cell is from genus *Corynebacterium,* genus *Brevibacterium,* genus *Arthrobacterium,* genus *Microbacterium,* or genus *Escherichia*; preferably, the host cell is *Corynebacterium glutamicum* or *Escherichia coli*; more preferably, the host cell is *Corynebacterium glutamicum* ATCC 13032, *Corynebacterium glutamicum* ATCC 13869, or *Corynebacterium glutamicum* ATCC 14067.
(7) Use of the polynucleotide according to (1) or (2), the transcription expression cassette according to (3), the recombinant expression vector according to (4), the recombinant host cell according to (5) or (6) in at least one of the following:
   (a) regulation of a transcriptional level of a gene, or preparation of a reagent or kit for regulating the transcriptional level of a gene;
   (b) preparation of a protein, or preparation of a reagent or kit for preparing a protein;
   (c) production of target compounds, or preparation of a reagent or kit for producing target compounds.
(8) The use according to (7), wherein the protein is selected from a gene expression regulatory protein or a protein involved in synthesis of a target compound.
(9) The use according to (7) or (8), wherein the target compound includes at least one of an amino acid or an organic acid; optionally, the amino acid includes at least one of lysine, glutamic acid, and threonine, the organic acid includes at least one of citric acid and succinic acid.
(10) A method for regulating the transcription of a target gene, wherein the method comprises a step of operably ligating the polynucleotide having promoter activity according to (1) or (2) with the target gene.
(11) A method for preparing a protein, comprising a step of expressing the protein using the transcription expression cassette according to (3), the recombinant expression vector according to (4), or the recombinant host cell according to (5) or (6); optionally, the protein is a protein involved in the synthesis of a target compound or a gene expression regulatory protein;
   optionally, the method further comprises a step of isolating or purifying the protein.
(12) A method for producing a target compound, comprising a step of expressing a protein involved in synthesis of a target compound or a gene expression regulatory protein using the transcription expression cassette of (3), the recombinant expression vector of (4), or the recombinant host cell of (5) or (6), and producing the target compounds in an environment where the protein involved in the synthesis of the target compound or the gene expression regulatory protein is present;
   optionally, the target compound includes at least one of an amino acid or an organic acid; optionally, the amino acid includes at least one of lysine, glutamic acid, and threonine, the organic acid includes at least one of citric acid and succinic acid;
   optionally, the protein is a protein involved in lysine synthesis; optionally, the protein involved in lysine synthesis includes one of or a combination of two or more of aspartate kinase, aspartate semialdehyde dehydrogenase, aspartate ammonia lyase, dihydrodipicolinate synthase, dihydropyridinic acid reductase, succinyl diaminopimelate aminotransferase, tetrahydrodipicolinate succinylase, succinyl diaminopimelate deacylase, diaminopimelic acid epimerase, diaminopimelic acid deacylase, glyceraldehyde-3-phosphate dehydrogenase, lysine transport protein, transketolase, diaminopimelate dehydrogenase, and pyruvate carboxylase;
   optionally, the method further comprises a step of isolating or purifying the target compounds.
(13) A polynucleotide having promoter activity, wherein the polynucleotide is any one selected from a group consisting of (v) to (x):
   (v) a mutant of the polynucleotide having the sequence as shown in SEQ ID NO: 1, the mutant having a mutated nucleotide at one or more positions corresponding to positions 170 to 179 of the sequence as shown in SEQ ID NO: 1; the activity of the mutant is higher than the promoter activity of the polynucleotide having the sequence as shown in SEQ ID NO: 1, and the nucleotide sequence of the mutant at positions corresponding to positions 170 to 179 in the sequence as shown in SEQ ID NO: 1 is not ACACCGAGTG;
   (vi) a mutant of a polynucleotide having the sequence as shown in SEQ ID NO: 2, the mutant having a mutated nucleotide at one or more positions corresponding to positions 130 to 139 in the sequence as shown in SEQ ID NO: 2; the activity of the mutant is higher than the promoter activity of the polynucleotide having the sequence as shown in SEQ ID NO: 2, and the nucleotide sequence of the mutant at positions corresponding to positions 130 to 139 in the sequence as shown in SEQ ID NO: 2 is not ACACCGAGTG;
   (vii) a mutant of a polynucleotide having the sequence as shown in SEQ ID NO: 3, the mutant having a mutated nucleotide at one or more positions corresponding to positions 72 to 81 in the sequence as shown in SEQ ID NO: 3; the activity of the mutant is higher than the promoter activity of the polynucleotide having the sequence as shown in SEQ ID NO: 3, and the nucleotide sequence of the mutant at positions corresponding to positions 72 to 81 in the sequence as shown in SEQ ID NO: 3 is not ACACCGAGTG;
   (viii) a polynucleotide having a reverse complementary sequence to the nucleotide sequence as set forth in any one of (v) to (vii);
   (ix) a polynucleotide having a reverse complementary sequence to a sequence that can be hybridized with the nucleotide sequence as set forth in any one of (i) to (vii) under high-stringent hybridization conditions or very high-stringent hybridization conditions;
   (x) a polynucleotide having a sequence of at least 80%, optionally at least 90%, preferably at least 95%, more preferably at least 97%, further preferably at least 98%, most preferably at least 99% sequence identity to the nucleotide sequence as set forth in any one of (v) to (vii).
(14) The polynucleotide having promoter activity according to (13), wherein the mutant has an enhanced promoter activity in an environment of increased salt concentration or osmolality;
   preferably, the mutant has 1 to 8 folds or more increased promoter activity in an environment of increased salt concentration or osmolality as compared with a polynucleotide having the sequence as shown in SEQ ID NO: 2.
(15) The polynucleotide having promoter activity according to (13) or (14), wherein the nucleotide sequence of the mutant at positions corresponding to positions 170 to 179 in the sequence as shown in SEQ ID NO: 2, or corresponding to positions 130 to 139 in the sequence as shown in SEQ ID NO: 3, or corresponding to positions 72 to 81 in the sequence as shown in SEQ ID NO: 4, is any one selected from a group consisting of (p₁) to (p₁₂):
   (p₁) TACTTGCAGA,
   (p₂) AGTGCTGAAA,
   (p₃) GCACGAAAGG,
   (p₄) TATCTAGAGG,
   (p₅) AGGCTTGTCG,
   (p₆) CGCTTCTTTC,
   (p₇) TAACTCTTGG
   (p₈) CCAAGTTCCA,
   (p₉) CGGTGCCACA,
   (p₁₀) AGCAGTTAGG,
   (p n) AGATAAATAA,
   (p₁₂) ATCGATCTAG
(16) The polynucleotide having promoter activity according to any one of (13) to (15), wherein a nucleotide sequence of the mutant is selected from the sequence as shown in any one of SEQ ID NOs: 37 to 48.
(17) A transcription expression cassette, wherein the transcription expression cassette comprises the polynucleotide having promoter activity according to any one of (13) to (16); optionally, the transcription expression cassette further comprises a target gene, the target gene is operably ligated to the polynucleotide having promoter activity; preferably, the target gene is a protein-coding gene.
(18) A recombinant expression vector, wherein the recombinant expression vector comprises the polynucleotide having promoter activity according to any one of (13) to (16), or the transcription expression cassette according to (17).
(19) A recombinant host cell, wherein the recombinant host cell comprises the transcription expression cassette according to (17), or the recombinant expression vector according to (18).
(20) The recombinant host cell according to (19), wherein the host cell is from genus *Corynebacterium,* genus *Brevibacterium,* genus *Arthrobacterium,* genus *Microbacterium,* or genus *Escherichia*; preferably, the host cell is *Corynebacterium glutamicum* or *Escherichia coli*; more preferably, the host cell is *Corynebacterium glutamicum* ATCC 13032, *Corynebacterium glutamicum* ATCC 13869, or *Corynebacterium glutamicum* ATCC 14067.
(21) Use of the polynucleotide having promoter activity according to (13) to (16), the transcription expression cassette according to (17), the recombinant expression vector according to (18), the recombinant host cell according to (19) or (20) in at least one of:
   (a) regulation of a transcriptional level of a gene, or preparation of a reagent or kit for regulating the transcriptional level of a gene;
   (b) preparation of a protein, or preparation of a reagent or kit for preparing a protein;
   (c) production of target compounds, or preparation of a reagent or kit for producing target compounds.
(22) The use according to (21), wherein the protein is selected from a gene expression regulatory protein or a protein involved in synthesis of a target compound.
(23) The use according to (21) or (22), wherein the target compound includes at least one of an amino acid or an organic acid; optionally, the amino acid includes at least one of proline, lysine, glutamic acid, threonine, glycine, alanine, valine, leucine, isoleucine, serine, cysteine, glutamine, methionine, aspartic acid, asparagine, arginine, histidine, phenylalanine, tyrosine, tryptophan; the organic acid includes at least one of citric acid, succinic acid, lactic acid, acetic acid, butyric acid, palmitic acid, oxalic acid, tartaric acid, propionic acid, hexenoic acid, capric acid, caprylic acid, valeric acid, malic acid.
(24) A method for regulating transcription of a target gene, wherein the method comprises operably ligating the polynucleotide having promoter activity according to any one of (13) to (16) with the target gene.
(25) A method for preparing a protein, wherein the method comprises expressing the protein using the transcription expression cassette according to (17), the recombinant expression vector according to (18), or the recombinant host cell according to (19) or (20); optionally, the protein is a protein involved in the synthesis of a target compound or a gene expression regulatory protein;
   optionally, the method further comprises a step of isolating or purifying the protein.
(26) A method for producing a target compound, wherein the method comprises a step of expressing a protein involved in the synthesis of a target compound or a gene expression regulatory protein using the transcription expression cassette according to (17), the recombinant expression vector according to (18), or the recombinant host cell according to (19) or (20), and producing target compounds in an environment where the protein involved in the synthesis of target compound or the gene expression regulatory protein is present;
   optionally, the target compound includes at least one of an amino acid or an organic acid; optionally, the amino acid includes at least one of lysine, glutamic acid, threonine, proline, glycine, alanine, valine, leucine, isoleucine, serine, cysteine, glutamine, methionine, aspartic acid, asparagine, arginine, histidine, phenylalanine, tyrosine, tryptophan, the organic acid includes at least one of citric acid, succinic acid, lactic acid, acetic acid, butyric acid, palmitic acid, oxalic acid, tartaric acid, propionic acid, hexenoic acid, capric acid, caprylic acid, valeric acid, malic acid;
   optionally, the protein involved in the synthesis of a target compound is a protein involved in the synthesis of L-amino acid; optionally, the protein involved in the synthesis of L-amino acid includes one of or a combination of two or more of pyruvate carboxylase, phosphoenolpyruvate carboxylase, γ-glutamyl kinase, glutamate semialdehyde dehydrogenase, pyrroline-5-carboxylate reductase, amino acid transport protein, ptsG system, pyruvate dehydrogenase, homoserine dehydrogenase, oxaloacetate decarboxylase, glucose-repressible protein, glucose dehydrogenase, aspartate kinase, aspartate semialdehyde dehydrogenase, aspartate ammonia lyase, dihydrodipicolinate synthase, dihydropyridinic acid reductase, succinyl diaminopimelate aminotransferase, tetrahydrodipicolinate succinylase, succinyl diaminopimelate deacylase, diaminopimelic acid epimerase, diaminopimelic acid deacylase, glyceraldehyde-3-phosphate dehydrogenase, transketolase, diaminopimelate dehydrogenase, or pyruvate carboxylase;
   optionally, the method further comprises a step of isolating or purifying the target compound.
(27) A polynucleotide having promoter activity, wherein the polynucleotide is any one selected from a group consisting of (xi) to (xiv):
   (xi) comprising a nucleotide sequence having the sequence as shown in any one of SEQ ID NOs:57 to 58;
   (xii) comprising a reverse complementary sequence to a nucleotide sequence having the sequence as shown in any one of SEQ ID NOs:57 to 58;
   (xiii) a reverse complementary sequence to a sequence that can be hybridized with the nucleotide sequence as set forth by (xi) or (xii) under high-stringent hybridization conditions or very high-stringent hybridization conditions, and having an enhanced promoter activity in an environment of increased salt concentration or osmolality,
   (xiv) a sequence having at least 99% sequence identity to the nucleotide sequence as set forth in (xi) or (xii), and having an enhanced promoter activity in an environment of increased salt concentration or osmolality.
(28) A transcription expression cassette, comprising the polynucleotide having promoter activity according to (27) and being operably ligated with a target gene.
(29) A recombinant expression vector, comprising the polynucleotide having promoter activity according to (27), or the transcription expression cassette according to (28).
(30) A recombinant host cell, comprising the transcription expression cassette according to (28), or the recombinant expression vector according to (29).
(31) The recombinant host cell according to (30), the host cell belonging to genus *Corynebacterium,* genus *Brevibacterium,* genus *Arthrobacterium,* genus *Microbacterium,* or genus *Escherichia.*
(32) The recombinant host cell according to (30), the host cell being *Corynebacterium glutamicum* ATCC 13032, *Corynebacterium glutamicum* ATCC 13869, or *Corynebacterium glutamicum* ATCC 14067, or a derivative strain of *Corynebacterium glutamicum.*
(33) A method for regulating transcription of a target gene, comprising a step of operably ligating the polynucleotide having promoter activity according to (27) with a target RNA or a target gene.
(34) A method according to (33), wherein the target RNA is at least one of tRNA, sRNA; the target gene comprises at least one of a coding gene of a protein involved in the synthesis of a target compound, a coding gene of a gene expression regulatory protein, or a coding gene of a protein involved in membrane transport.
(35) A method for preparing a protein, comprising a step of expressing a target protein using the transcription expression cassette according to (28), the recombinant expression vector according to (29), or the recombinant host cell according to any one of (30) to (32).
(36) The method according to (35), wherein the target protein is a protein involved in the synthesis of a target compound, a protein involved in membrane transport, or a gene expression regulatory protein.
(37) The method according to (35) or (36), further comprising a step of isolating or purifying the target protein.
(38) A method for producing a target compound, comprising a step of expressing a protein involved in the synthesis of a target compound, a protein involved in membrane transport, or a gene expression regulatory protein using the transcription expression cassette according to (28), the recombinant expression vector according to (29), or the recombinant host cell according to any one of (30) to (32), producing target compounds in an environment where the protein involved in the synthesis of the target compound, the protein involved in membrane transport or the gene expression regulatory protein is present.
(39) The method according to (38), wherein the target compounds is at least one of an amino acid or an organic acid.
(40) The method according to (39), wherein the amino acid is one of or a combination of two or more of proline, hydroxyproline, lysine, glutamic acid, threonine, glycine, alanine, valine, leucine, isoleucine, serine, cysteine, glutamine, methionine, aspartic acid, asparagine, arginine, histidine, phenylalanine, tyrosine, tryptophan, 5-aminolevulinic acid, or derivatives of any one of the above amino acids.
(41) The method according to (38) or (39), wherein the protein involved in the synthesis of the target compound is one of or a combination of two or more of pyruvate carboxylase, phosphoenolpyruvate carboxylase, γ-glutamyl kinase, glutamate semialdehyde dehydrogenase, pyrroline-5-carboxylate reductase, amino acid transport protein, ptsG system, pyruvate dehydrogenase, homoserine dehydrogenase, oxaloacetate decarboxylase, glucose-repressible protein, glucose dehydrogenase, aspartate kinase, aspartate semialdehyde dehydrogenase, aspartate ammonia lyase, dihydrodipicolinate synthase, dihydrodipicolinate reductase, dihydropyridinic acid reductase, succinyl diaminopimelate aminotransferase, tetrahydrodipicolinate succinylase, succinyl diaminopimelate deacylase, diaminopimelic acid epimerase, diaminopimelic acid deacylase, glyceraldehyde-3-phosphate dehydrogenase, transketolase, diaminopimelate dehydrogenase.
(42) The method according to (39), the organic acid is one of or a combination of two or more of citric acid, succinic acid, lactic acid, acetic acid, butyric acid, palmitic acid, oxalic acid, oxaloacetic acid, tartaric acid, propionic acid, hexenoic acid, capric acid, caprylic acid, valeric acid, malic acid, or derivatives of any one of the above organic acids.
(43) The method according to any one of (38) to (42), further comprising a step of isolating or purifying the target compounds.
(44) Use of the polynucleotide having promoter activity according to (27), the transcription expression cassette according to (28), the recombinant expression vector according to (29), the recombinant host cell according to any one of (30) to (32) in at least one of the following:
   a. regulation of a transcriptional level of a gene, or preparation of a reagent or kit for regulating the transcriptional level of a gene;
   b. preparation of a protein, or preparation of a reagent or kit for preparing a protein;
   c. production of a target compound, or preparation of a reagent or kit for producing a target compound.

### Effects

In one embodiment, the present disclosure provides a polynucleotide having promoter activity, which is a high salt and high osmolality-inducible promoter and has an enhanced promoter activity in an environment of increased salt concentration and osmolality. The polynucleotide being operably ligated with a target gene can significantly increase the expression intensity of the target gene in a stress environment of high salt and high osmolality, enabling stable and efficient production of downstream products, effectively solving the existing problem of adding expensive inducers such as IPTG and causing toxicity to the strain.

In another embodiment, the present disclosure provides a transcription expression cassette, a recombinant expression vector, and a recombinant host cell, which comprise the aforesaid polynucleotide having promoter activity. In the transcription expression cassette, the recombinant expression vector, and the recombinant host cell, the polynucleotide having promoter activity is operably ligated with a protein-coding gene, improving the expression intensity of the protein-coding gene in a stress environment of high salt and high osmolality.

In another embodiment, the present disclosure provides a method for producing amino acids, using the aforesaid polynucleotide having promoter activity, which can increase the expression of the proteins involved in the synthesis of amino acids in a stress environment while weakening the expression of the proteins of other pathways, so that the metabolic flux concentrates more towards the synthesis of amino acids, thereby producing amino acids in a stable and efficient manner to achieve excessive accumulation of amino acids.

In another embodiment, when the aforesaid method is used to produce L-lysine, it can stably and efficiently produce L-lysine in an environment of high salt and high osmolality.

In some embodiments, the polynucleotide having promoter activity provided by the present disclosure is a mutant obtained based on a polynucleotide having the sequence shown in any one of SEQ ID NOs: 1 to 3. The mutant of the present disclosure has an enhanced promoter activity in an environment of increased salt concentration or osmolality and is a high salt and high osmolality-inducible promoter. As compared with the inducible promoters such as *tac*, *trc*, the mutant of the present disclosure does not require IPTG, which is expensive and has certain toxicity, as the inducer. As compared with the wild-type promoter, the mutant of the present disclosure has a further enhanced promoter activity in a high salt and high osmolality environment. By operably ligating the mutant to a target gene, it can improve the expression efficiency of the target gene, thereby stably and efficiently producing target compounds. Further, in the high salt or high osmolality inducement environment, the conversion rate of the mutant in the production of target compounds is higher than the conversion rate of the wild-type promoter, thereby providing an inducible promoter that is very promising in the application of industrial fermentation for target compounds such as amino acids and organic acids. In some embodiments, the mutant of a polynucleotide having the sequence as shown in any one of SEQ ID NOs: 1 to 3 according to the present disclosure has 1 to 8 folds or more increased promoter activity in an environment of increased salt concentration or osmolality as compared with the wild-type promoter.

In some more specific embodiments, the mutant of the polynucleotide having the sequence as shown in any one of SEQ ID NOs: 1 to 3 provided by the present disclosure has 1.85 to 7.62 folds increased promoter activity in an environment of increased salt concentration or osmolality as compared with the wild-type promoter.

In some embodiments, the present disclosure provides a transcription expression cassette, a recombinant expression vector, a recombinant host cell, which comprises the aforesaid polynucleotide having promoter activity. In the transcription expression cassette, the recombinant expression vector, and the recombinant host cell, the polynucleotide having promoter activity is operably ligated with a target gene, increasing the expression intensity of key genes in the synthesis pathways for the target compounds in a stress environment of high salt and high osmolality.

In some embodiments, the present disclosure provides a method for preparing a protein which is capable of increasing an expression amount of a protein involved in the synthesis of amino acids, organic acids, and the like, or an expression amount of a gene expression regulatory protein, thereby achieving efficient production of target compounds.

In some embodiments, the present disclosure provides a method for producing target compounds, which, by using the polynucleotide having promoter activity, can enhance the expression of a protein involved in the synthesis of target compounds in a stress environment, and thus stably and efficiently produce the target compounds, fulfilling the purpose of increasing the metabolic fluxes of the target compounds and excessive accumulation of the target compounds.

### BRIEF DESCRIPTION OF THE DRAWINGS

Fig. 1 illustrates the inducing effect of high osmolality on the NCgl1418 promoter;
Fig. 2 illustrates the inducing effect of sodium sulfate of different concentrations on the NCgl1418 promoter;
Fig. 3 illustrates the inducing effect of a high concentration of saccharides on the NCgl1418 promoter;
Fig. 4 illustrates a comparison result of the promoter intensities of P*_{NCgl1418}* and P*_{tuf}*;
Fig. 5 illustrates a comparison result of the activities of NCgl1418 promoters at different lengths;
Fig. 6 illustrates the intensities and high osmolality-induced activities of the *proP* promoter and the promoter mutants;
Fig. 7 illustrates a comparison result of the activities of the *proP* promoter and the NCgl1418 promoter.

### DETAILED DESCRIPTION

When used in combination with the term "comprising" in the claims and/or specification, the word "a" or "an" may refer to "one", or refer to "one or more", "at least one", and "one or more than one".

As used in the claims and specification, the term "comprise", "have/has", "include", or "contain" is intended to be inclusive or open-ended, and does not exclude additional or unrecited elements or methods and steps.

Throughout the application document, the term "about" means that a value includes the error or standard deviation caused by the device or method used to measure the value.

It is applicable to the content disclosed herein that the term "or" is defined only as alternatives and "and/or", but the term "or" used herein refers to "and/or" unless otherwise expressly stated to be merely alternatives or mutual exclusion between alternatives.

The selected/optional/preferred "numerical range", when used in the claims or the specification, includes not only the numerical endpoints at both ends of the range, but also all natural numbers covered between the preceding numerical endpoints with respect to these numerical endpoints.

As used in the present disclosure, the term "high salt environment" refers to a high concentration of inorganic salt ions such as Na₂SO₄, NaCl, K₂SO₄, KCl in the culture media, or an increased concentration of the products such as lysine or intermediate metabolites (e.g., lysine sulfate) in the fermentation broth due to accumulation as the fermentation time extends, or an increased concentration due to the substrate feeding (e.g., a substrate of ammonium sulfate), or a concentration of any salt that may exist in the fermentation broth. In some specific embodiments, the term "high-salt environment" relates to a salt concentration above 0.2M; in some more specific embodiments, the term "high salt environment" relates to a salt concentration of 0.2M to 0.8M, for example a salt concentration of 0.2M, 0.3M, 0.4M, 0.5M, 0.6M, 0.7M, 0.8M.

As used the present disclosure, the term "high osmolality environment" refers to an increased osmolality in response to an increased salt concentration.

As used herein, the term "polynucleotide" refers to a polymer composed of nucleotides. A polynucleotide may be in the form of an individual fragment, or may be a constituent part of a larger nucleotide sequence structure, which is derived from the nucleotide sequence that has been isolated at least once in number or concentration, and can be recognized, operated, and restored from the sequence and its component nucleotide sequences by standard molecular biology methods (e.g., using a cloning vector). When a nucleotide sequence is represented by a DNA sequence (i.e., A, T, G, C), this also includes an RNA sequence (i.e., A, U, G, C), where "U" substitutes for "T". In other words, "polynucleotide" refers to a nucleotide polymer deleted from an additional nucleotide (an individual fragment or an entire fragment), or may be a constituent part or component of a larger nucleotide structure, such as an expression vector or a polycistronic sequence. Polynucleotides include DNA, RNA, and cDNA sequences.

As used herein, the terms "sequence identity" and "percent identity" refer to the percentage of nucleotides or amino acids that are the same (i.e., identical) between two or more polynucleotides or polypeptides. The sequence identity between two or more polynucleotides or polypeptides may be determined by aligning the nucleotide sequences of polynucleotides or the amino acid sequences of polypeptides and scoring the number of positions at which nucleotide or amino acid residues are identical in the aligned polynucleotides or polypeptides, and comparing the number of these positions with the number of positions at which nucleotide or amino acid residues are different in the aligned polynucleotides or polypeptides. Polynucleotides may differ at one position by, e.g., containing a different nucleotide (i.e., substitution or mutation) or deleting a nucleotide (i.e., insertion or deletion of a nucleotide in one or two polynucleotides). Polypeptides may differ at one position by, e.g., containing a different amino acid (i.e., substitution or mutation) or deleting an amino acid (i.e., insertion or deletion of an amino acid in one or two polypeptides). The sequence identity may be calculated by dividing the number of positions at which nucleotide or amino acid residues are identical by the total number of nucleotide or amino acid residues in the polynucleotides or polypeptides. For example, the percent identity may be calculated by dividing the number of positions at which nucleotide or amino acid residues are identical by the total number of nucleotide or amino acid residues in the polynucleotides or polypeptides, and multiplying the result by 100.

In some specific embodiments, the polynucleotide having promoter activity has a reverse complementary sequence to the nucleotide sequence as shown in the sequence of any one of SEQ ID NOs: 1 to 3, 57 to 58, and the polynucleotide remains high salt and high osmolality-inducible promoter activity.

In some specific embodiments, the polynucleotide having promoter activity comprises a reverse complementary sequence to a sequence that can be hybridized with a nucleotide sequence as shown in a sequence of any one of SEQ ID NOs: 1 to 3, 57 to 58 or a reverse complementary sequence thereto under high-stringent hybridization conditions or very high-stringent hybridization conditions, and the polynucleotide maintains a high salt and high osmolality-inducible promoter activity.

In some specific embodiments, the polynucleotide having promoter activity comprises a sequence having at least 80%, 81%, 82%, 83%, 84%, 85%, 86%, 87%, 88%, 89%, 90%, 91%, 92%, 93%, 94%, 95%, 96%, 97%, 98%, 99%, 100% sequence identity to any one of the afore-described nucleotide sequences, and the polynucleotide maintains a high salt and high osmolality-inducible promoter activity.

As used herein, the term "wild-type" refers to an object that can be found in nature. For example, a polypeptide or a polynucleotide sequence present in an organism that can be isolated from a natural source and has not been intentionally modified by human in the laboratory is naturally occurring. As used herein, "naturally occurring" and "wild-type" are synonymous. In some embodiments, the wild-type promoter mentioned in the present disclosure refers to the wild-type promoter of *NCgl1418*, i.e., the polynucleotide having the sequence as shown in SEQ ID NO: 1.

The term "mutant" used herein refers to a polynucleotide or polypeptide including alternation(s) (i.e., substitution, insertion and/or deletion) at one or more (e.g., several) positions, as compared with the "wild-type" or "comparative" polynucleotide or polypeptide. Here, substitution refers to a substitution of a different nucleotide or amino acid for a nucleotide or amino acid that occupies one position; deletion refers to removal of a nucleotide or amino acid that occupies some position; and insertion refers to an addition of a nucleotide or amino acid after the nucleotide or amino acid adjacent to and immediately following the occupied position.

In some embodiments, the term "mutation" used in the present disclosure means "substitution" which is a mutation caused by a substitution of one base in one or more nucleotide with another different base, which is also referred to as a base substitution or a point mutation.

Specifically, the sequence as shown in SEQ ID NO: 36 is the core region sequence of the promoter of *NCgl1418*, including the main sequences of the -35 region and the -10 region. The term "mutation" used in the present disclosure refers to a mutated nucleotide introduced at a position in the vicinity of the -35 region. It is discovered that the mutant with the mutation introduced at the above position has a significantly enhanced promoter activity in a high salt or high osmolality environment.

In some embodiments, a mutant of the polynucleotide having the sequence as shown in SEQ ID NO: 1 refers to the mutant of the polynucleotide having the sequence as shown in SEQ ID NO: 1 which has a mutated nucleotide at one or more positions corresponding to positions 170 to 179 of the sequence as shown in SEQ ID NO: 1 and does not include the polynucleotide mutated to ACACCGAGTG at positions corresponding to positions 170 to 179 of the sequence as shown in SEQ ID NO: 1. As compared with the polynucleotide having the sequence as shown in SEQ ID NO: 1, the mutant has an increased promoter activity. In some more specific embodiments, as compared with the polynucleotide having the sequence as shown in SEQ ID NO: 1, the mutant has an increased promoter activity in an environment of increased salt concentration and osmolality.

In some embodiments, a mutant of the polynucleotide having the sequence as shown in SEQ ID NO: 2 refers to the mutant of the polynucleotide having the sequence as shown in SEQ ID NO: 2 which has a mutated nucleotide at one or more positions corresponding to positions 130 to 139 in the sequence as shown in SEQ ID NO: 2 and does not include the polynucleotide mutated to ACACCGAGTG at positions corresponding to positions 130 to 139 of the sequence as shown in SEQ ID NO: 2. As compared with the polynucleotide having the sequence as shown in SEQ ID NO: 2, the mutant has an increased promoter activity. In some more specific embodiments, as compared with the polynucleotide having the sequence as shown in SEQ ID NO: 2, the mutant has an increased promoter activity in an environment of increased salt concentration and osmolality.

In some embodiments, a mutant of a polynucleotide having the sequence as shown in SEQ ID NO: 3 refers to a mutant of a polynucleotide having the sequence as shown in SEQ ID NO: 3 which has a mutated nucleotide at one or more positions corresponding to positions 72 to 81 of the sequence shown in SEQ ID NO: 3 and does not include the polynucleotide mutated to ACACCGAGTG at positions corresponding to positions 72 to 81 of the sequence as shown in SEQ ID NO: 3. As compared with the polynucleotide having the sequence as shown in SEQ ID NO: 3, the mutant has an increased promoter activity. In some more specific embodiments, as compared with the polynucleotide having the sequence as shown in SEQ ID NO: 3, the mutant has an increased promoter activity in an environment of increased salt concentration and osmolality.

In some embodiments, as compared with the polynucleotide having the sequence as shown in SEQ ID NO: 1, the mutant according to the present disclosure has 1 to 8 folds or more increased promoter activity in an environment of increased salt concentration or osmolality.

Further, as compared with the polynucleotide having the sequence as shown in SEQ ID NO: 1, the mutant has 2.11, 1.85, 3.23, 3.07, 3.57, 2.78, 3.90, 3.28, 2.62, 2.70, 4.47, 7.62 folds increased promoter activity in an environment of increased salt concentration and osmolality.

As used herein, the term "high-stringent conditions" means that following the standard DNA blotting procedures, a probe of at least 100 nucleotides in length pre-hybridizes or hybridizes for 12 to 24 hours at 42°C in 5X SSPE (saline sodium phosphate EDTA), 0.3% SDS, 200 µg/ml of cleaved and denatured salmon sperm DNA, and 50% formamide. Finally, the vector material is washed three times at 65°C with 2X SSC and 0.2% SDS, each for 15 min.

As used herein, the term "very high-stringent conditions" means that following the standard DNA blotting procedures, a probe of at least 100 nucleotides in length pre-hybridizes or hybridizes for 12 to 24 hours at 42°C in 5X SSPE (saline sodium phosphate EDTA), 0.3% SDS, 200 µg/ml of cleaved and denatured salmon sperm DNA, and 50% formamide. Finally, the vector material is washed three times at 70°C with 2X SSC and 0.2% SDS, each for 15 min.

As used herein, the term "complementary" refers to hybridization or base pairing between nucleotides or nucleic acids, e.g., between two chains of a double-stranded DNA molecule or between an oligonucleotide primer and a primer binding site on a sequenced or amplified single-stranded nucleotide.

In the present disclosure, the term "promoter" refers to a nucleic acid molecule, which is typically located upstream of the coding sequence of the target gene, providing a recognition site for RNA polymerase, and is located upstream in the 5' direction of the transcription initiation site of mRNA. It is an untranslated nucleic acid sequence to which RNA polymerase binds and which initiates transcription of the target gene. In the synthesis of ribonucleic acid (RNA), the promoter can interact with transcription factors and regulate gene transcription and control the initiation time and degree of gene expression (transcription), and it comprises a core promoter region and regulatory region, functions like a "switch", determines the activity of the gene and then controls which kind of protein the cell starts to produce.

In the present disclosure, the term "promoter core region" refers to a nucleic acid sequence located in the promoter region of prokaryotes, and it is the core sequence region that plays the role of promoter, mainly including the -35 region, the -10 region, and the region between the -35 region and the -10 region, and the transcription initiation site, the -35 region being the recognition site of RNA polymerase, and the -10 region being the binding site of RNA polymerase.

In some specific embodiments, the polynucleotide having promoter activity of the present disclosure can be used to initiate the expression of protein-coding genes. In some other examples, the polynucleotide having promoter activity of the present disclosure can be used to initiate the expression of non-coding genes.

In the present disclosure, the term "expression" includes any step involving RNA production and protein production, including but not limited to transcription, post-transcriptional modification, translation, post-translational modification and secretion.

The term "target gene" according to the present disclosure refers to any gene that is ligated to the polynucleotide having promoter activity of the present disclosure to regulate a transcriptional level of the polynucleotide having promoter activity of the present disclosure.

In some embodiments, the term "target gene" refers to a gene encoding a target protein of microorganisms. Exemplarily, the target gene is a gene encoding an enzyme involved in the biosynthesis of the target compounds, a gene encoding an enzyme involved in reducing power, a gene encoding an enzyme involved in glycolysis or the TCA cycle, or a gene encoding an enzyme involved in releasing target compounds, etc.

The "target compound" used in the present disclosure is selected from at least one of an amino acids and an organic acids or selected from compounds of other types that can be obtained through biosynthesis in this field.

In some embodiments, the target compound is "amino acid" or "L-amino acid". The term "amino acid" or "L-amino acid" usually refers to a basic constituting unit of protein of which the amino group and the carboxy group are bond to the same carbon atom. Exemplarily, the amino acid selected from one of or a combination of two or more of glycine, alanine, valine, leucine, isoleucine, threonine, serine, cysteine, glutamine, methionine, aspartic acid, asparagine, glutamic acid, lysine, arginine, histidine, phenylalanine, tyrosine, tryptophan, 5-aminolevulinic acid, proline, or amino acids of other types in this field.

In some embodiments, the target compound is an organic acid. The organic acid could be an organic compound having acidity, such as a compound comprising a carboxyl group or a sulfonic acid group. Exemplarily, the organic acid includes one of or a combination of two or more of lactic acid, acetic acid, succinic acid, butyric acid, palmitic acid, oxalic acid, tartaric acid, citric acid, propionic acid, hexenoic acid, capric acid, caprylic acid, valeric acid, oxaloacetic acid, malic acid, or organic acids of other types in the art.

In the present disclosure, the term "protein-coding gene" refers to a DNA molecule capable of directing the synthesis of a protein by certain rules, and the process by which a protein-coding gene directing protein synthesis generally includes transcription process using double-stranded DNAs a template and translation using mRNAs a template. The protein-coding gene contains CDS sequence (Coding Sequence) directing the production of protein-coding mRNA.

Exemplarily, the protein-coding gene includes, but is not limited to, those being used to encode a protein involved in synthesis of target compounds. Exemplarily, the protein-coding gene relates to encoding a protein involved in the synthesis of L-amino acid. The protein involved in the synthesis of L-amino acid includes, but is not limited to, one of or a combination of two or more of pyruvate carboxylase, phosphoenolpyruvate carboxylase, γ-glutamyl kinase, glutamate semialdehyde dehydrogenase, pyrroline-5-carboxylate reductase, amino acid transport protein, ptsG system, pyruvate dehydrogenase, homoserine dehydrogenase, oxaloacetate decarboxylase, glucose-repressible protein, glucose dehydrogenase. In some embodiments, the protein-coding gene relates to coding a protein involved in synthesis of L-lysine. The protein involved in synthesis of L-lysine includes one of or a combination of two or more of aspartate kinase, aspartate semialdehyde dehydrogenase, aspartate ammonia lyase, dihydrodipicolinate synthase, dihydropyridinic acid reductase, succinyl diaminopimelate aminotransferase, tetrahydrodipicolinate succinylase, succinyl diaminopimelate deacylase, diaminopimelic acid epimerase, diaminopimelic acid deacylase, glyceraldehyde-3-phosphate dehydrogenase, lysine transport protein, transketolase, diaminopimelate dehydrogenase, and pyruvate carboxylase.

In some embodiments, the protein-coding gene is for encoding a protein involved in synthesis of organic acids. Exemplarily, the protein-coding gene is for encoding a protein involved in synthesis of citric acid, or for encoding a protein involved in synthesis of succinic acid.

In some other examples, the protein-coding gene is for encoding a protein involved in gene editing, such as a Cpf1 protein. The polynucleotide having promoter activity according to the present disclosure is suitable for enhancing expression of a target gene in a stress environment of high salt and high osmolality, thereby achieving efficient production of target products.

The term "gene expression regulatory protein" used in the present disclosure includes, but is not limited to, a protein of an exogenous gene expression regulation tool, such as dCas9 required for CRISPRi regulation, dCpf1, Hfq required for sRNA regulation, etc., as well as endogenous or exogenous transcriptional regulation factors and regulates expression of key genes in the metabolism pathway.

In the present disclosure, the term "transcription expression cassette" refers to a kind of expression element comprising a transcription regulatory element and a target gene, and uses the transcription regulatory element to regulate the expression of the target gene. In the present disclosure, the transcription regulatory element comprises a promoter, and may further comprise elements such as an enhancer, a silencer, an insulator. In the present disclosure, the target gene is specifically a protein-coding gene. "Operably ligating" a target gene to a polynucleotide means that a polynucleotide having promoter activity is functionally ligated with a target gene to initiate and mediate the transcription of the target gene, and the operably ligating can be in any of the ways described by those skilled in the art.

In the present disclosure, the term "vector" refers to a DNA construct containing a DNA sequence operably ligated with suitable regulatory sequences, so as to express a target gene in a suitable host. "Recombinant expression vector" refers to a DNA structure used to express, for example, a polynucleotide coding a desired polypeptide. The recombinant expression vector may include, for example, i) a collection of genetic elements that have regulatory functions on gene expression, such as promoters and enhancers; ii) a structure or coding sequence transcribed into mRNA and translated into a protein; and iii) transcription subunits which appropriately transcribe and translate the start and stop sequences. The recombinant expression vector is constructed in any suitable way. The property of the vector is not critical, and any vector can be used, including plasmids, viruses, phages and transposons. Possible vectors for use in the present disclosure include, but are not limited to, chromosomal, non-chromosomal and synthetic DNA sequences, such as bacterial plasmids, phage DNA, yeast plasmids and vectors derived from combinations of plasmids and phage DNA, and DNA from viruses such as vaccinia virus, adenovirus, fowl pox virus, baculovirus, SV40 and pseudorabies virus, etc.

In the present disclosure, the term "host cell" refers to any cell type that is easily transformed, transfected, transduced, etc., with a transcription initiation element or expression vector comprising the polynucleotide of the present disclosure. The term "recombinant host cell" covers a host cell that differs from the parent cell after introduction of a transcription initiation element or a recombinant expression vector, and the recombinant host cell is specifically realized by transformation.

In the present disclosure, the term "transformation" has the meaning generally understood by those skilled in the art, i.e., the process of introducing exogenous DNA into a host. The transformation method includes any method for introducing nucleic acid into a cell, including but not limited to electroporation, calcium phosphate precipitation, calcium chloride (CaCl₂) precipitation, microinjection, polyethylene glycol (PEG) method, DEAE-dextran method, cationic liposome method and lithium acetate-DMSO method.

The host cell of the present disclosure can be a prokaryotic cell or an eukaryotic cell, as long as it is a cell to which the polynucleotide having promoter activity of the present disclosure can be introduced. In one embodiment, the host cell refers to a prokaryotic cell. Specifically, the host cell is derived from a microorganism suitable for producing amino acids by fermentation, such as genus *Corynebacterium,* genus *Brevibacterium,* genus *Arthrobacterium,* genus *Microbacterium* or genus *Escherichia.* Preferably, the host cell is *Corynebacterium glutamicum* from genus *Corynebacterium.* The *Corynebacterium glutamicum* can be *Corynebacterium glutamicum* ATCC 13032, *Corynebacterium glutamicum* ATCC 13869, *Corynebacterium glutamicum* ATCC 14067, and the like, and a mutant strain producing amino acids which is prepared from the above strains or a derivative strain of *Corynebacterium glutamicum.* In some embodiments, the host cell of the present disclosure may be any types of strain capable of producing the target product, which includes wild-type strains and recombinant strains.

Exemplarily, the host cell is a lysine-producing host cell. In some embodiments, the lysine-producing host cell may be a strain expressing aspartate kinase that relieves feedback inhibition based on *Corynebacterium glutamicum* ATCC 13032. In addition, the lysine-producing host cell may be a strain of other types that is capable of producing lysine.

In some embodiments, one or more genes selected from the following are attenuated or reduced in expression in the lysine-producing host cell:
a. *adhE* gene encoding ethanol dehydrogenase;
b. *ackA* gene encoding acetate kinase;
c. *pta* gene encoding phosphate acetyltransferase;
d. *ldhA* gene encoding lactic dehydrogenase;
e. *focA* gene encoding formate transporter;
f. *pflB* gene encoding pyruvate formate lyase;
g. *poxB* gene encoding pyruvate oxidase;
h. *thrA* gene encoding aspartate kinase I/homoserine dehydrogenase I bifunctional enzyme;
i. *thrB* gene encoding homoserine kinase;
j. *ldcC* gene encoding lysine decarboxylase; and
h. *cadA* gene encoding lysine decarboxylase.

In some embodiments, one or more genes selected from the following are enhanced or overexpressed in the lysine-producing host cell:
a. *dapA* gene encoding dihydrodipyridine synthase that relieves feedback inhibition of lysine;
b. *dapB* gene encoding dihydrodipicolinate reductase;
c. *ddh* gene encoding diaminopimelate dehydrogenase;
d. *dapD* coding tetrahydrodipicolinate succinylase and dapE coding succinyl diaminopimelate deacylase;
e. *asd* gene encoding aspartate-semialdehyde dehydrogenase;
f. *ppc* gene encoding phosphoenolpyruvate carboxylase;
g. *pntAB* gene encoding nicotinamide adenine dinucleotide transhydrogenase;
i. *lysE* gene encoding lysine transport protein.

The host cell is a threonine-producing host cell. In some embodiments, the threonine-producing host cell is a strain expressing the aspartate kinase *LysC* gene that relieves feedback inhibition on the basis of *Corynebacterium glutamicum* ATCC 13032. In some other embodiments, the threonine-producing host cell may be a strain of other types that is capable of producing threonine.

In some embodiments, one or more genes selected from the following are enhanced or overexpressed in the threonine-producing host cell:
a. *thr ABC* gene encoding threonine operon;
b. *hom* gene encoding homoserine dehydrogenase that relieves feedback inhibition;
c. *gap* gene encoding glyceraldehyde-3-phosphate dehydrogenase;
d. *pyc* gene encoding pyruvate carboxylase;
e. *mqo* gene encoding malate: quinone oxidoreductase;
f. *tkt* gene encoding transketolase;
g. *gnd* gene encoding 6-phosphogluconate dehydrogenase;
h. *thrE* gene encoding threonine exporter;
i. *eno* gene encoding enolase.

Exemplarily, the host cell is an isoleucine-producing host cell. In some embodiments, the isoleucine-producing host cell is a strain producing L-isoleucine by substituting alanine for the amino acid at position 323 of the *ilvA* gene of L-threonine dehydratase.

In some other embodiments, the isoleucine-producing host cell may be a strain of other types that is capable of producing isoleucine.

Exemplarily, the host cell is an O-acetylhomoserine-producing host cell. In some embodiments, the O-acetylhomoserine-producing host cell is a strain that produces O-acetylhomoserine by inactivating O-acetylhomoserine (thiol)-lyase. In some other embodiments, the O-acetylhomoserine-producing host cell may be a strain of other types that is capable of producing O-acetylhomoserine.

Exemplarily, the host cell is a methionine-producing host cell. In some embodiments, the methionine-producing host cell is a strain that produces methionine by inactivating transcription regulators of methionine and cysteine. In some other embodiments, the methionine-producing host cell may be a strain of other types that is capable of producing methionine.

The culture of the host cell of the present disclosure may be performed by a conventional method, including but not limited to: well-plate culture, shaking culture, batch culture, continuous culture, fed-batch culture, etc., with various culture conditions such as temperature, time, and pH value of the medium being properly adjusted suitably according to actual situations.

The polynucleotide having promoter activity of the present disclosure is a self-inducible promoter and exhibits an enhanced promoter activity in a high salt and high osmolality environment, avoiding adding an expensive and toxic inducer such as IPTG, which is commonly used in this field, to the fermentation environment.

Unless otherwise defined in the present disclosure or clearly indicated in the background, all technical and scientific terms used in the present disclosure have the same meaning as usually understood by an ordinary skilled person in the field of the present disclosure.

### High salt and high osmolality-inducible promoter

In the present disclosure, *Corynebacterium glutamicum* ATCC 13032 strains are cultured in CGXII culture media to which 0.6 M NaCl or lysine sulfate is added or not added. Cells are collected in the mid-log phase of growth; the total RNA is extracted and RNA-sequencing analysis performed. A promoter of the *NCgl1418* gene having obvious increase in the transcriptional level under high osmolality conditions is selected as the top candidate high salt and high osmolality-inducible promoter.

In some specific embodiments, the NCgl1418 promoter is any one selected from a group consisting of (i) to (iv):
(i) a nucleotide sequence having a sequence as shown in any one of SEQ ID NOs: 1 to 3;
(ii) a reverse complementary sequence to a nucleotide sequence having the sequence as shown in any one of SEQ ID NOs: 1 to 3;
(iii) a reverse complementary sequence to a sequence that can be hybridized with the nucleotide sequence as set forth in (i) or (ii) under high-stringent hybridization conditions or very high-stringent hybridization conditions;
(iv) a sequence having at least 80%, 81%, 82%, 83%, 84%, 85%, 86%, 87%, 88%, 89%, 90%, 91%, 92%, 93%, 94%, 95%, 96%, 97%, 98%, 99% sequence identity to the nucleotide sequence as set forth in (i) or (ii);

In some specific embodiments, the NCgl1418 promoter has an enhanced promoter activity in a high salt environment. In some specific embodiments, the NCgl1418 promoter has an enhanced promoter activity in a high osmolality environment.

As used in the present disclosure, the term "high salt environment" refers to a high concentration of inorganic salt ions such as Na₂SO₄, NaCl, K₂SO₄, KCl in the culture media, or an increased concentration of the products such as lysine or intermediate metabolites (e.g., lysine sulfate) in the fermentation broth due to accumulation as the fermentation time extends, or an increased concentration due to the substrate feeding (e.g., a substrate of ammonium sulfate), or a concentration of any salt that may exist in the fermentation broth. In some specific embodiments, the term "high salt environment" relates to a salt concentration above 0.2M; in some more specific embodiments, the term "high salt environment" relates to a salt concentration of 0.2M to 0.8M, for example a salt concentration of 0.2M, 0.3M, 0.4M, 0.5M, 0.6M, 0.7M, 0.8M.

As used the present disclosure, the term "high osmolality environment" refers to an increased osmolality in response to an increased salt concentration.

In some preferable examples, the NCgl1418 promoter exhibits an enhanced promoter activity or a higher conversion rate in a "high salt environment" formed by sulfate or hydrochloride. In some preferable examples, the sulfate is Na₂SO₄ or K₂SO₄, or lysine sulfate; the hydrochloride is lysine hydrochloride, sodium chloride, or potassium chloride.

In some preferable examples, the promoter having the nucleotide sequence as shown in the sequence of any one of SEQ ID NOs: 57 to 58exhibits an enhanced promoter activity or a higher conversion rate in a "high salt environment" formed with a sulfate. In some preferable examples, the sulfate is Na₂SO₄ or K₂SO₄.

In some specific embodiments, the polynucleotide having promoter activity comprises a reverse complementary sequence to the nucleotide sequence as shown in the sequence of any one of SEQ ID NOs: 57 to 58, and the polynucleotide maintains a high salt and high osmolality-inducible promoter activity.

In some specific embodiments, the polynucleotide having promoter activity comprises a reverse complementary sequence to a sequence that can be hybridized with the nucleotide sequence as shown in the sequence of any one of SEQ ID NOs: 57 to 58 or a reverse complementary sequence thereto under high-stringent hybridization conditions or very high-stringent hybridization conditions, and the polynucleotide maintains a high salt and high osmolality-inducible promoter activity.

In some specific embodiments, the polynucleotide having promoter activity has a sequence having at least 80%, 81%, 82%, 83%, 84%, 85%, 86%, 87%, 88%, 89%, 90%, 91%, 92%, 93%, 94%, 95%, 96%, 97%, 98%, 99%, 100% sequence identity to any one of the afore-described sequences, and the polynucleotide maintains a high salt and high osmolality-inducible promoter activity.

### Mutant having the promoter core region of the NCgl1418 gene

In the present disclosure, mutation is introduced into the promoter of the *NCgl1418* gene upstream the -35 region using the sequence of the promoter core region of the *NCgl1418* gene, to obtain a mutant having a mutated promoter core region of the *NCgl1418* gene upstream the -35 region.

Specifically, mutation is introduced into the ACACCGAGTG upstream the -35 region of the promoter core region of the *NCgl1418* gene, obtaining a mutant with a further enhanced promoter activity in a high salt or high osmolality environment, thereby providing a novel inducible promoter for genetic engineering and breeding of fermenting microorganisms and efficient production of target compounds. As compared with the wild-type promoter of the *NCgl1418* gene, the mutant of the present disclosure exhibits an enhanced promoter activity in an environment of increased salt concentration or osmolality. When applied to fermentation for target compounds, the mutant exhibits a higher conversion rate to target compounds as compared with the wild-type promoter.

In addition, by truncating the promoter to different lengths, fragments of *NCgl1418* promoter having 203 bp (SEQ ID NO: 2) and 145 bp (SEQ ID NO: 3) are obtained, respectively. Both fragments have the core region of the *NCgl1418* promoter and exhibit obviously enhanced promoter activities in an environment of increased salt concentration and osmolality. Therefore, by the promoter modification method of the afore-described examples i.e. by mutating one or more positions corresponding to positions 130 to 139 of the sequence as shown in SEQ ID NO: 2 or mutating one or more positions corresponding to positions 72 to 81 of the sequence as shown in SEQ ID NO: 3, a promoter mutant of the same enhanced induced activity is obtained.

In the present disclosure, the term "high salt environment" refers to a high concentration of inorganic salt ions such as Na₂SO₄, NaCl, K₂SO₄, KCl in the culture media, or an increased concentration of the products such as lysine or intermediate metabolites (e.g., lysine sulfate) in the fermentation broth due to accumulation as the fermentation time extends, or an increased concentration due to the substrate feeding (e.g., a substrate of ammonium sulfate), or a concentration of any salt that may exist in the fermentation broth.

In some embodiments, a mutant of the polynucleotide having the sequence as shown in SEQ ID NO: 1 refers to the mutant of the polynucleotide having the sequence as shown in SEQ ID NO: 1 which has a mutated nucleotide at positions corresponding to one or more of positions 170 to 179 of the sequence as shown in SEQ ID NO: 1 and does not include a polynucleotide mutated to ACACCGAGTG at the positions corresponding to positions 170 to 179 of the sequence as shown in SEQ ID NO: 1. As compared with the polynucleotide having the sequence as shown in SEQ ID NO: 1, the mutant has an enhanced promoter activity. In some more specific embodiments, as compared with the polynucleotide having the sequence as shown in SEQ ID NO: 1, the mutant has an enhanced promoter activity in an environment of increased salt concentration and osmolality.

In some embodiments, the mutant has a mutated nucleotide at 1, 2, 3, 4, 5, 6, 7, 8, 9, or 10 positions corresponding to positions 170 to 179 of the sequence as shown in SEQ ID NO: 1.

In some embodiments, a mutant of the polynucleotide having the sequence as shown in SEQ ID NO: 2 refers to the mutant of the polynucleotide having the sequence as shown in SEQ ID NO: 2 which has a mutated nucleotide at one or more positions corresponding to positions 130 to 139 of the sequence as shown in SEQ ID NO: 2 and does not include the polynucleotide mutated to ACACCGAGTG at the positions corresponding to positions 130 to 139 of the sequence as shown in SEQ ID NO: 2. As compared with the polynucleotide having the sequence as shown in SEQ ID NO: 2, the mutant has an enhanced promoter activity. In some more specific embodiments, as compared with the polynucleotide having the sequence as shown in SEQ ID NO: 2, the mutant has an enhanced promoter activity in an environment of increased salt concentration and osmolality.

In some embodiments, the mutant has a mutated nucleotide at 1, 2, 3, 4, 5, 6, 7, 8, 9, or 10 positions corresponding to positions 130 to 139 of the sequence as shown in SEQ ID NO: 2.

In some embodiments, a mutant of the polynucleotide having the sequence as shown in SEQ ID NO: 3 refers to the mutant of the polynucleotide having the sequence as shown in SEQ ID NO: 3 which has a mutated nucleotide at one or more positions corresponding to positions 72 to 81 of the sequence as shown in SEQ ID NO: 3 and does not include the polynucleotide mutated to ACACCGAGTG at the positions corresponding to positions 72 to 81 of the sequence as shown in SEQ ID NO: 3. As compared with the polynucleotide having the sequence as shown in SEQ ID NO: 3, the mutant has an enhanced promoter activity. In some more specific embodiments, as compared with the polynucleotide having the sequence as shown in SEQ ID NO: 3, the mutant has an enhanced promoter activity in an environment of increased salt concentration and osmolality.

In some embodiments, the mutant has a mutated nucleotide at 1, 2, 3, 4, 5, 6, 7, 8, 9, or 10 positions corresponding to positions 72 to 81 of the sequence as shown in SEQ ID NO: 3.

In some embodiments, the polynucleotide having promoter activity of the present disclosure further comprises a polynucleotide in a complementary direction to the nucleotide sequence of the mutant of the promoter of the *NCgl1418* gene.

In some embodiments, the polynucleotide having promoter activity of the present disclosure further comprises a polynucleotide that is reverse complementary to a sequence comprising or hybridized with the mutant of the promoter of *NCgl1418* under high-stringent hybridization conditions or very high-stringent hybridization conditions. Further, the nucleotide sequence of the polynucleotide is not ACACCGAGTG at the positions corresponding to positions 170 to 179 of the sequence as shown in SEQ ID NO: 1; the nucleotide sequence of the polynucleotide is not ACACCGAGTG at the positions corresponding to positions 130 to 139 of the sequence as shown in SEQ ID NO: 2; the nucleotide sequence of the polynucleotide is not ACACCGAGTG at the positions corresponding to positions 72 to 81 of the sequence as shown in SEQ ID NO: 3.

In some embodiments, the polynucleotide having promoter activity of the present disclosure has at least 80%, 81%, 82%, 83%, 84%, 85%, 86%, 87%, 88%, 89%, 90%, 91%, 92%, 93%, 94%, 95%, 96%, 97%, 98%, 99% (including all ranges and percentages between these numbers) sequence identity to the sequence of the afore-described polynucleotide. Moreover, the nucleotide sequence of the polynucleotide is not ACACCGAGTG at the positions corresponding to positions 170 to 179 of the sequence as shown in SEQ ID NO: 1, the nucleotide sequence of the polynucleotide is not ACACCGAGTG at the positions corresponding to positions 130 to 139 of the sequence as shown in SEQ ID NO: 2, the nucleotide sequence of the polynucleotide is not ACACCGAGTG at the positions corresponding to positions 72 to 81 of the sequence as shown in SEQ ID NO: 3.

In some specific embodiments, a nucleotide sequence of the mutant at the positions corresponding to positions 170 to 179 of the sequence as shown in SEQ ID NO: 1, at the positions corresponding to positions 130 to 139 of the sequence as shown in SEQ ID NO: 2, and at the positions corresponding to positions 72 to 81 of the sequence as shown in SEQ ID NO: 3 is any one selected from a group consisting of (p₁) to (p₁₂): (p₁) TACTTGCAGA, (p₂) AGTGCTGAAA, (p₃) GCACGAAAGG, (p₄) TATCTAGAGG, (p₅) AGGCTTGTCG, (p₆) CGCTTCTTTC, (p₇) TAACTCTTGG, (p₈) CCAAGTTCCA, (p₉) CGGTGCCACA, (p₁₀) AGCAGTTAGG, (p₁₁) AGATAAATAA, (p₁₂) ATCGATCTAG

In some specific embodiments, the nucleotide sequence of the mutant is selected from a sequence as shown in any one of SEQ ID NOs: 37 to 48.

In some embodiments, the polynucleotide having promoter activity of the present disclosure has 1 to 8 folds or more increased promoter activity as compared with the polynucleotide having the sequence as shown in SEQ ID NO: 1 and has 2.11, 1.85, 3.23, 3.07, 3.57, 2.78, 3.90, 3.28, 2.62, 2.70, 4.47, 7.62 folds increased promoter activity in an environment of increased salt concentration or osmolality as compared with the polynucleotide having the sequence as shown in SEQ ID NO: 1.

### Construction of recombinant expression vector and recombinant host cell

In some specific embodiments of the present disclosure, with the ATCC 13032 genome as a template, a promoter sequence (SEQ ID NO: 1) of the NCgl1418 gene was obtained through PCR amplification using the primers 1418-F and 1418-R; using pXM-*gfp* as a template, amplification was performed on pXM-*gfp* using the primers pGFP-F and pGFP-R to obtain vector fragments from which the *ladI* gene and the tac promoter were deleted; the above fragments were recombinantly ligated to obtain the recombinant pXM-P_{NCgl1418}-*gfp* expression vectors.

In some specific embodiments of the present disclosure, using pXM-P*_{NCgl1418}*-*gfp* as a template, fragments of the NCgl1418 promoter respectively having 203 bp, 145 bp, and 94 bp were obtained through PCR amplification using the primer pairs 1418-203-F/R, 1418-145-F/R, and 1418-94-F/R; the three fragments were recovered and phosphorylated by T4 PNK; new vectors were obtained by self-ligation construction and named as pXM-P₂₀₃-*gfp*, pXM-P₁₄₅-*gfp*, and pXM-P₉₄-*gfp*, respectively.

In some specific embodiments of the present disclosure, with the ATCC 13032 genome as a template, the promoter sequence of the NCgl1418 gene was obtained through PCR amplification using the primers 1418-D-F and 1418-D-R. Using pXM-07 as a template, first, the vector fragment 1 having dCpf1 was obtained through PCR amplification using the primers pXM07-F1 and pXM07-R1; then, the vector fragment 2 having an origin of replication was obtained through PCR amplification using the primers pXM07-F2 and pXM07-R2; using pEC-26 as a template, the crRNA array fragment targeting *gltA, pgi, hom*, and *pck* genes was obtained through PCR amplification using the primers pEC26-F and pEC26-R. The above fragments were recovered and recombinantly ligated to obtain the recombinant pXM-P*_{NCgl1418}*-dCpf1 expression vectors.

In some specific embodiments of the present disclosure, with the ATCC 13032 genome as a template, the promoter sequence of the NCgl1418 gene and the DNA sequence of the *lysE* gene were obtained through PCR amplification using the primers 1418-L-F and 1418-L-R. Using pEC-XK99E as a template, vector fragments were obtained through PCR amplification using the primers pEC-F and pEC-R. The above three fragments were recovered and recombinantly ligated to obtain the recombinant pEC-P_{NCgl1418}-*lysE* expression vectors.

The *Corynebacterium glutamicum* is specifically from *Corynebacterium glutamicum ATCC 13032* (the sequence of ATCC 13032 genome: NC_003450.3). In some embodiments of the present disclosure, using pXM-P*_{NCgl1418}*-*gfp* as a template, reverse PCR amplification was performed using the primers 1418mutant-F and 1418mutant-R on pXM-P*_{NCgl1418}*-*gfp* to obtain linearized plasmid fragments; the linearized plasmid fragments were phosphorylated and ligated, and resistance clones were collected, to obtain a library of mutant promoters of the *NCgl1418* gene.

In some embodiments of the present disclosure, the *Corynebacterium glutamicum* ATCC13032 was transfected with the library of mutant promoters of the NCgl1418 gene, pXM-Con, and pXM-P*_{NCgl1418}*-*gfp*, respectively, to obtain recombinant host cells. Fluorescence intensities of the recombinant host cells were screened after culturing on a plate, to screen mutants with enhanced promoter intensity.

In some specific embodiments of the present disclosure, using pXM-P*_{NCgl1418}*-dCpf1 as a template, PCR amplification was performed using the primers 35P7-F and pXM-R, and the primers pXM-F and 35P7-R1, respectively, to obtain two vector fragments. These fragments were recombinantly ligated to obtain the recombinant pXM-P_{35P7}-dCpf1 vector.

In some specific embodiments of the present disclosure, using pEC-P*_{NCgl1418}*-*lysE* as a template, the vector fragment having the 35P7 promoter mutant and having the *lysE* gene was obtained through PCR amplification using the primers 35P7-F and 35P7-R2; then, the linearized vector fragment was phosphorylated, and pEC-P_{35P7}-*lysE* was obtained through self-ligation construction.

In some other embodiments of the present disclosure, a sequence of the promoters 35P1, 35P2, 35P3, 35P4, 35P5, 35P6, 35P8, 35P9, 35P10, 35P11, 35P12 may be used to construct the required recombinant vector in view of the specific cloning requirements.

In some specific embodiments of the present disclosure, with the ATCC 13032 genome as a template, the promoter sequence of the proP gene (SEQ ID NO: 56) were obtained through PCR amplification using the primers proP-F and proP-R; using pXM-*gfp* as a template, pXM-*gfp* was amplified using the primers pGFP-F and pGFP-R to obtain vector fragments from which the *lacI* gene and the tac promoter were deleted; the above fragments were recombinantly ligated to obtain the recombinant pXM-P*_{proP}*-*gfp* expression vector.

In some specific embodiments of the present disclosure, using pXM-P_{proP}-gfp as a template, fragments obtained through PCR amplification using the primers proP-1-F and proP-1-R were phosphorylated by T4 PNK, and pXM-P_{proP-1}-gfp was obtained through self-ligation construction. Meanwhile, using pXM-P_{proP}-gfp as a template, fragments obtained through PCR amplification using the primers proP-2-F and proP-2-R were phosphorylated by T4 PNK, and pXM-Pₚᵣₒₚ₋₂-gfp was obtained through self-ligation construction.

In some specific embodiments of the present disclosure, with the ATCC 13032 genome as a template, the sequence of the P*_{proP}* promoter was obtained through PCR amplification using the primers proP-D-F and proP-D-R. Using pXM-07 as a template, first, the vector fragment having dCpf1 was obtained through PCR amplification using the primers pXM07-F1 and pXM07-R2; then, the vector fragment 2 having an origin of replication was obtained through PCR amplification using the primers pXM07-F2 and pXM07-R2; using pEC-26 as a template, the crRNA array fragment targeting the *gltA*, *pgi*, *hom*, and *pck* genes was obtained through PCR amplification using the primers pEC26-F and pEC26-R; the above fragments were recovered and recombinantly ligated to obtain the recombinant pXM-P*_{proP}*-dCpf1 expression vector.

In some specific embodiments of the present disclosure, using pXM-P*_{proP-1}*-gfp as a template, primers proP-D-F and proP1-D-R were designed, and fragments of the P*_{proP-1}* promoter were obtained through PCR amplification. Using pXM-07 as a template, first, the vector fragment 1 having dCpf1 was obtained through PCR amplification using primers pXM07-F1 and pXM07-R2; then, the vector fragment 2 having an origin of replication was obtained through PCR amplification using the primers pXM07-F2 and pXM07-R2; using pEC-26 as a template, the crRNA array fragments targeting the *gltA*, *pgi*, *hom*, and *pck* genes were obtained through PCR amplification using the primers pEC26-F and pEC26-R; the above fragments were recovered and recombinantly ligated to obtain the recombinant pXM-P*_{proP-1}*-dCpf1 expression vector.

In some specific embodiments of the present disclosure, using pXM-P_{proP}-gfp as a template, fragments of the P_{proP-1} promoter sequence were obtained through PCR amplification using the primers proP-lysE-F and proP-lysE-R; using pXM-P_{proP-1}-gfp as a template, fragments of the P_{proP} promoter sequence were obtained through PCR amplification using the primers proP-lysE-F and proP1-lysE-R. With the ATCC 13032 genome as a template, fragments of the *lysE* gene were obtained through PCR amplification using the primers lysE-F and lysE-R. Meanwhile, using pXM-XK99E as a template, vector fragments were obtained through PCR amplification using the primers pEC-F and pEC-R. The above promoter fragments were recombinantly ligated to the *lysE* fragments and the vector fragments, respectively, and the ligated products were transfected into Trans T1 competent cells, coated on a Kanamycin resistance plate and cultured overnight; positive clones were picked for colony PCR verification, the correct transformants were subjected to sequence confirmation, the obtained recombinant vectors were named pEC-P_{proP}-lysE and pEC-P_{proP-1}-lysE. In some embodiments, the *Corynebacterium glutamicum* SCgL30 strain in the present disclosure is a SCgL30 strain having certain lysine-synthesis capability obtained through construction by mutating a threonine at position 311 of the aspartate kinase (encoded by *lysC* gene) in the *Corynebacterium glutamicum* ATCC13032 genome to isoleucine.

In some embodiments of the present disclosure, pEC-P_{35P7}-*lysE* was transfected into recombinant SCgL30 strains, to obtain the recombinant host cells. In some embodiments of the present disclosure, pXM-P_{35P7}-dCpf1 was transfected into the recombinant SCgL30 strains, to obtain the recombinant host cells. In some other embodiments of the present disclosure, the recombinant vectors respectively comprising the sequences of the promoters 35P1, 35P2, 35P3, 35P4, 35P5, 35P6, 35P8, 35P9, 35P10, 35P11, 35P12 may be transfected into the recombinant SCgL30 strains, to obtain the recombinant host cells.

### Process of producing target compounds

(1) operably ligating the polynucleotide having promoter activity to a gene encoding a protein involved in the synthesis of target compounds or a gene encoding a gene expression regulatory protein, to obtain a recombinant expression vector capable of expressing the protein involved in synthesis of target compounds or the gene expression regulatory protein, transfecting the recombinant expression vector into the host cell, obtaining a recombinant host cell;
(2) fermentation culturing the recombinant host cell, collecting target compounds from the culturing liquor of the recombinant host cell or the recombinant host cell, completing the production of the target compounds.

During the above production process, because of the improved promoter activity of the polynucleotide, in the recombinant host cell, the transcriptional activity of the protein involved in synthesis of target compounds or the gene encoding the gene expression regulatory protein increases, the amount of expression of the protein involved in synthesis of target compounds or the gene expression regulatory protein increases, thereby significantly increasing the yield of the target compounds.

In some specific embodiments, the steps of the method used by the present disclosure for preparing amino acids do not include addition of an inducer. In a specific example of the present disclosure, the steps of the method used by the present disclosure for preparing amino acids do not include addition of IPTG.

In some embodiments, the target compound is an amino acid; the gene encoding a protein involved in the synthesis of target compounds refers to a gene encoding a protein involved in the synthesis of amino acid. In some embodiments, the target compound is L-amino acid; the gene encoding a protein involved in the synthesis of amino acids refers to a gene encoding a protein involved in the synthesis of L-amino acid. In some specific embodiments, the L-amino acid is L-lysine, the gene encoding a protein involved in the synthesis of amino acids is the lysine transport protein LysE. By increasing the expression of LysE with the polynucleotide having promoter activity, it is possible to facilitate exocytosis and extracellular accumulation of lysine. In some embodiments, the gene expression regulatory protein is dCpf1. dCpf1 is capable of targeted regulation for target genes such as *gltA*, *pgi*, *hom*, and *pck.* The polynucleotide having promoter activity can enhance the expression of dCpf1 in a high salt environment, thereby increasing the weakened degree of the target genes, further facilitating the synthesis of lysine and the utilization of the substrate.

In some specific embodiments, the host cell is *Corynebacterium glutamicum. Corynebacterium glutamicum* is an important strain for producing L-lysine. After being reconstructed with the high salt and high osmolality-inducible polynucleotide, transcription expression cassette or recombinant expression vector, *Corynebacterium glutamicum* has a significantly increased amount of expression of the proteins involved in the synthesis of lysine, specifically a significantly increased amount of expression in a high salt and high osmolality environment, thereby dramatically improving the capability of *Corynebacterium glutamicum* of L-lysine fermentation accumulation in a long period of time.

In some specific embodiments, the host cell is the following modified *Corynebacterium glutamicum*: the threonine at position 311 of the aspartate kinase (encoded by the *lysC* gene) in the *Corynebacterium glutamicum* ATCC13032 genome is mutated to isoleucine.

In some specific embodiments, the culture conditions for the recombinant host cells were as follows: the recombinant host cells were inoculated into a TSB liquid medium containing a corresponding antibiotic, cultured overnight at 30°C, 220 r/min, and with the initial OD 0.3, respectively transferred to lysine fermentation media to which 0.6M sodium sulfate was added or not added (simulating the high salt high osmolality environment due to high-concentration product accumulation in the later phase of fermentation); the culture system was a 24-well plate containing 1 mL/well in each well; the fermentation was terminated after culturing for 36 h at 30°C, 800 r/min, and glucose residue, OD₆₀₀, and lysine yield were measured.

Ingredients of the lysine fermentation media were as follows: glucose, 80 g/L; yeast powder, 8 g/L; urea, 9 g/L; K₂HPO₄, 1.5 g/L; MOPS, 42 g/L; FeSO₄, 0.01 g/L; MnSO₄, 0.01 g/L; MgSO₄, 0.6 g/L; the final chloramphenicol concentration being 5 µg/mL, and/or the final kanamycin concentration being 25 µg/mL.

In some specific embodiments, recovering the target compounds from the recombinant host cells or the culture solution of the recombinant host cells may be performed by a common method in this field, including but not limited to: filtration, anion exchange chromatography, crystallization, and HPLC.

In this field, the method for manipulating microorganisms is known, as explained in the publications such as Current Protocols in Molecular Biology (Online ISBN: 9780471142720, John Wiley and Sons, Inc.), Microbial Metabolic Engineering: Methods and Protocols (Qiong Cheng Ed., Springer), and Systems Metabolic Engineering: Methods and Protocols (Hal S. Alper Ed., Springer).

### Examples

Additional objectives, features, and advantages of the present disclosure are apparent from the subsequent description in detail. However, it is to be appreciated that the detailed description and specific embodiments (although indicating specific embodiments of the present disclosure) are provided merely for illustrative purpose. Based on the detailed description, various changes and modifications within the spirits and the scopes of the present disclosure will become apparent to a person skilled in the art.

The experimental techniques and experimental methods used in the examples, unless otherwise specified, are all conventional techniques and methods, for example, experimental methods for which no specific conditions are indicated in the following examples are generally performed according to conventional conditions such as those described by Sambrook et al., Molecular Cloning: A Laboratory Manual (New York: Cold Spring Harbor Laboratory Press, 1989), or those recommended by manufacturers. The materials, reagents, etc. used in the examples, unless otherwise specified, are all commercially available.

The sequences of the primers used in the construction of plasmids of the examples are shown in Table 1 below.

**Table 1**

| Primers | Sequence (5'-3') | SEQ ID NO |
|---|---|---|
| 1418-F | CTTTTCACCAGTGAGACGGGTAAAACTCGCGATGAAGT AG | SEQ ID NO:6 |
| 1418-R | GTTCTTCTCCTTTACTCATCATTGGCCTTTCTCGAATTG GG | SEQ ID NO:7 |
| pGFP-F | ATGAGTAAAGGAGAAGAACTTTTCAC | SEQ ID NO:8 |
| pGFP-R | CCCGTCTCACTGGTGAAAAG | SEQ ID NO:9 |
| tuf-F | CACCAGTGAGACGGGAGATCGTTTAGATCCGAAGGAA AA | SEQ ID NO:10 |
| tuf-R | CTCGAATTGGGTATCAACGGACTTCGTGGTGGCTACGA | SEQ ID NO:11 |
| tuf-pGFP-F | GTTGATACCCAATTCGAGAAAGG | SEQ ID NO:12 |
| tuf-pGFP-R | CCCGTCTCACTGGTGAAAAG | SEQ ID NO:13 |
| 1418-203-F | GACACCTGTGAGTTTCAAACTCC | SEQ ID NO:14 |
| 1418-203-R | CCCGTCTCACTGGTGAAAAG | SEQ ID NO:15 |
| 1418-145-F | TTTGTTTATGCAGGTGGCG | SEQ ID NO:16 |
| 1418-145-R | CCCGTCTCACTGGTGAAAAG | SEQ ID NO:17 |
| 1418-94-F | GTTTCTATGTATTAAAGATCACACCG | SEQ ID NO:18 |
| 1418-94-R | CCCGTCTCACTGGTGAAAAG | SEQ ID NO:19 |
| 1418-D-F | CTTTTCACCAGTGAGACGGGTAAAACTCGCGATGAAGT AG | SEQ ID NO:20 |
| 1418-D-R | TTCTTGATAAATTGACACCATTGGCCTTTCTCGAATTGG G | SEQ ID NO:21 |
| pXM07-F1 | GTGTCAATTTATCAAGAATTTGTTAATAAA | SEQ ID NO:22 |
| pXM07-R1 | GCGGATACATATTTGAATGTATTTAG | SEQ ID NO:23 |
| pXM07-F2 | ACACGCGTCTGAGCAGTATTCATGAGACAATAACCCTG ATAAATGC | SEQ ID NO:24 |
| pXM07-R2 | CCCGTCTCACTGGTGAAAAG | SEQ ID NO:25 |
| pEC26-F | CATTCAAATATGTATCCGCGAGAGTCAATTCAGGGTGG TG | SEQ ID NO:26 |
| pEC26-R | AATACTGCTCAGACGCGTGTC | SEQ ID NO:27 |
| pXM07-F3 | GTGTCAATTTATCAAGAATTTGTTAATAAA | SEQ ID NO:28 |
| pXM07-R3 | CCCGTCTCACTGGTGAAAAG | SEQ ID NO:29 |
| 1418-L-F | AGCGGCATGCATTTACGTTTAAAACTCGCGATGAAGTA G | SEQ ID NO:30 |
| 1418-L-R | AGATTTCCATGATCACCATCATTGGCCTTTCTCGAATTG GG | SEQ ID NO:31 |
| lysE-F | ATGGTGATCATGGAAATCTTCATTAC | SEQ ID NO:32 |
| lysE-R | GTCTGTTTCCTGTGTGAAACTAACCCATCAACATCAGT TTGATG | SEQ ID NO:33 |
| pEC-F | TTTCACACAGGAAACAGACCATG | SEQ ID NO:34 |
| pEC-R | AACGTAAATGCATGCCGCTTC | SEQ ID NO:35 |
| 1418mutant-F | NNNNNNNNNNGTGGAATTTCCTCAAGTGATTTACC | SEQ ID NO:49 |
| 1418mutant-R | GATCTTTAATACATAGAAACAGATCTGTTACCC | SEQ ID NO:50 |
| 35P7-F | TAACTCTTGGGTGGAATTTCCTCAAGTGATTTAC | SEQ ID NO:51 |
| 35P7-R1 | AAATTCCACCCAAGAGTTAGATCTTTAATACATAGAAA CAGATCTGTTACC | SEQ ID NO:52 |
| 35P7-R2 | GATCTTTAATACATAGAAACAGATCTGTTACC | SEQ ID NO:53 |
| pXM-F | CAAAGGCGGTAATACGGTTATC | SEQ ID NO:54 |
| pXM-R | AACCGTATTACCGCCTTTGAG | SEQ ID NO:55 |
| proP-F | TTTCACCAGTGAGACGGGGCACCGAAAACAGTAACTT TCC | SEQ ID NO:59 |
| proP-R | TTCTTCTCCTTTACTCATGAGTAAAACCTCTCGTCATAT CTTATGAG | SEQ ID NO:60 |
| proP-1-F | CGAGAAAGGCCAATGATGAGTAAAGGAGAAGAACTTT TCAC | SEQ ID NO:61 |
| proP-1-R | AATTGGGTATCAAGTGCATAACGGAATGTTTGTTTG | SEQ ID NO:62 |
| proP-2-F | ACAATGGACTTTGTTGATACCCAATTCGAGAAAGGCCA ATGATGAGTAAAGGAGAAGAACTTTTCAC | SEQ ID NO:63 |
| proP-2-R | GGGTAAATCACTTGAGGAAATTCCACCACTCGGTGTGA TCTTTAATATGGAAGAATGCAAAAAGACCC | SEQ ID NO:64 |
| proP-D-F | TTTTCACCAGTGAGACGGGGCACCGAAAACAGTAACT TTCC | SEQ ID NO:65 |
| proP-D-R | AATTCTTGATAAATTGACACGAGTAAAACCTCTCGTCAT ATCTTATGAG | SEQ ID NO:66 |
| proP1-D-R | ATTCTTGATAAATTGACACCATTGGCCTTTCTCGAATTG G | SEQ ID NO:67 |
| proP-lysE-F | GCGGCATGCATTTACGTTGCACCGAAAACAGTAACTTT CC | SEQ ID NO:68 |
| proP-lysE-R | AAGATTTCCATGATCACCATGAGTAAAACCTCTCGTCAT ATCTTATGAG | SEQ ID NO:69 |
| proP1-lysE-R | AGATTTCCATGATCACCATCATTGGCCTTTCTCGAATTG G | SEQ ID NO:70 |

### Example 1. Screening of inducible promoters

The *Corynebacterium glutamicum* ATCC 13032 strains were cultured in CGXII culture media to which 0.6 M NaCl or lysine sulfate was added or not added; cells were collected in the mid-log phase of growth; the total RNA was extracted and RNA-sequencing analysis performed. Promoters of the *NCgl1418* gene having an obvious increase in the transcriptional level under high osmolality conditions were selected as the top candidate high salt and high osmolality-inducible promoters.

### Example 2. Construction of recombinant vectors having the promoter sequence

According to the genomic sequence (NC _003450.3) of *Corynebacterium glutamicum* ATCC 13032 published by NCBI, the primers 1418-F (SEQ ID NO: 6) and 1418-R (SEQ ID NO: 7) were designed. With the ATCC 13032 genome as a template, the promoter sequence (SEQ ID NO: 1) of the *NCgl1418* gene was obtained through PCR amplification, parameters of the PCR amplification being as follows: 95°C 5 min, 95°C 30 s, 65-55°C 30 s, 72°C 1 min, repeating 10 times, 95°C 30 s, 55°C 30 s, 72°C 1 min, repeating 25 times, 72°C with 10 min extension. Meanwhile, with the pXM-*gfp* reported in the literature as a template^{[10]}, vector fragments from which the *lacI* gene and the tac promoter were deleted were obtained through PCR amplification using the primers pGFP-F and pGFP-R, parameters of the PCR amplification being as follows: 95°C 10 min, 95°C 30 s, 65-55°C 30 s, 72°C 3 min, repeating 10 times, 95°C 30 s, 55°C 30 s, 72°C 3 min, repeating 25 times, 72°C with 10 min extension. After being recovered, the above two fragments were recombinantly ligated via Vazyme Clon Express Multies One Step Cloning Kit, and the ligated product was transfected into the Trans T1 competent cells, coated on a chloramphenicol resistance plate and cultured overnight; positive clones were picked for colony PCR verification, the correct transformants were subjected to sequence confirmation, the obtained recombinant vector was named pXM-P*_{NCgl1418}*-*gfp*. Moreover, the vector fragments were phosphorylated by T4 PNK, and the control pXM-con vector was obtained through self-ligation construction.

### Example 3. Inducing effect of high salt on the NCg11418 promoter

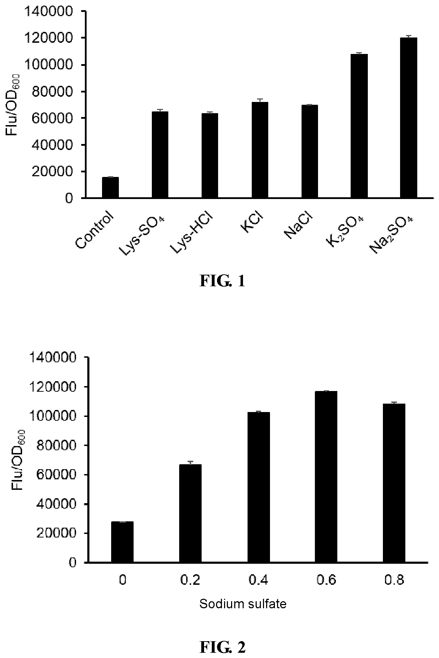

The recombinant pXM-P*_{NCgl1418}*-*gfp* vector and the control pXM-con vector were respectively transfected into *Corynebacterium glutamicum* ATCC 13032 to obtain recombinant strains and control strains. These strains were respectively inoculated into TSB culture media containing 5 µg/mL chloramphenicol and cultured overnight at 30°C, 220 r/min. The ingredients of the TSB liquid medium were as follows (g/L): glucose, 5 g/L; yeast powder, 5 g/L; soy peptone, 9 g/L; urea, 3 g/L; succinic acid, 0.5 g/L; K₂HPO₄•3H₂O, 1 g/L; MgSO₄•7H₂O, 0.1 g/L; biotin, 0.01 mg/L; vitamin B1, 0.1 mg/L; MOPS, 20 g/L. With the initial OD 0.5, the strains were respectively transferred to CGXIIY culture media to which 0.6 M of different salts were added or not added, the culture system being a 24-well plate containing 1 mL/well of culture media. After culturing for 18h at 30°C, 800 r/min, GFP fluorescence intensity and OD₆₀₀ were measured for different strains; relative intensities of the NCgl1418 promoter under different conditions were characterized by the fluorescence intensity per strain cell (subtracting the fluorescence intensity per strain cell of the control strain under the same conditions). The ingredients of the CGXIIY culture media were as below: glucose,50 g/L; NH₄Cl 16.5 g/L; urea 5 g/L; KH₂PO₄ 1 g/L; K₂HPO₄ 1 g/L; MOPS 42 g/L; MgSO₄ 0.25 g/L; FeSO₄·2H₂O 0.01 g/L; MnSO₄·H₂O 0.01 g/L; ZnSO₄·7H₂O 0.001 g/L; CuSO₄ 0.2 mg/L; NiCl·6H₂O 0.02 mg/L; CaCl₂ 0.01 g/L; protocatechuic acid, 0.03 g/L; biotin, 0.2 mg/L; Vitamin B1, 0.1 mg/L; the final chloramphenicol concentration being 5 µg/mL. The measurement results are shown in Fig. 1; the data shows that the conversion rates of the NCgl1418 promoter and the expression of the report gene can be induced (4.1 to 7.7 folds) by adding 0.6 M of different salts; thus, it was determined that the promoter was inducible by high salt.

### Example 4. Inducing effect of different osmolality on the NCgl1418 promoter

By the method of Example 3, the inducing effects of different concentrations of sodium sulfate on the NCgl1418 promoter were measured. The results are shown in Fig. 2; the data shows that the intensities of the NCgl1418 promoter are enhanced as the concentrations of sodium sulfate increases, exhibiting obvious gradient induced activities within a certain range.

### Example 5. Response of the NCgl1418 promoter to high saccharide

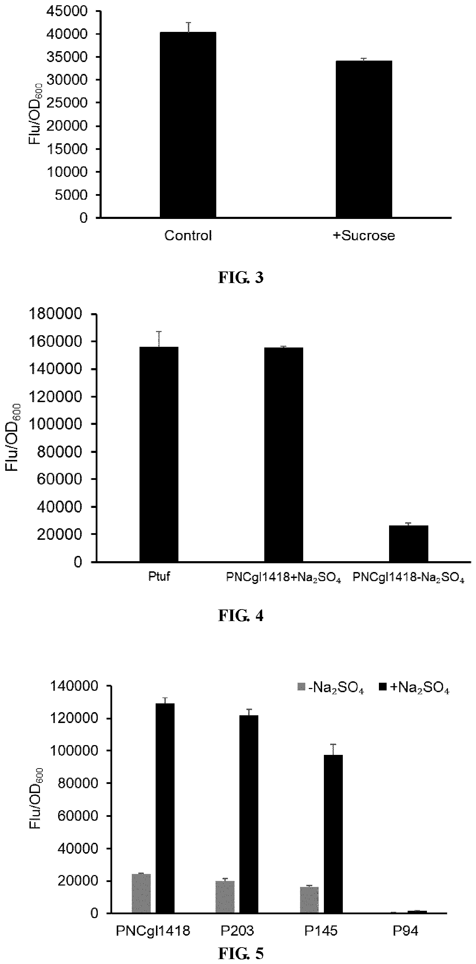

By the method of Example 3, the inducing effect of high concentration of sucrose (150 g/L) on the NCgl1418 promoter was measured. The result is shown in Fig. 3; the data shows that further addition of high-concentration sucrose had no obvious effect of enhancing the induction activity of the NCgl1418 promoter, which means that the promoter does not respond to all high osmolality stresses, but responds to high-concentration salt ions specifically.

### Example 6. Intensity comparison between the NCgl1418 promoter and the tuf promoter

At present, the known and commonly used endogenous strong promoter of *Corynebacterium glutamicum* is P*_{tuf}*. Therefore, a comparison between the NCgl1418 promoter and the P*_{tuf}* promoter was performed to verify the intensity of the NCgl1418 promoter. According to the genomic sequence (NC _003450.3) of *Corynebacterium glutamicum* ATCC 13032 published by NCBI, the primers tuf-F (SEQ ID NO: 10) and tuf-R (SEQ ID NO: 11) were designed. With the ATCC 13032 genome as a template, a promoter sequence (SEQ ID NO: 5) having the *tuf* gene was obtained through PCR amplification, the parameters of the PCR amplification being as follows: 95°C 5 min, 95°C 30 s, 55°C 30 s, 72°C 1 min, repeating 30 times, 72°C with 10 min extension. Meanwhile, using pXM-P*_{NCgl1418}*-*gfp* as a template, vector fragments comprising RBS of *P_{NCgl1418}* were obtained through PCR amplification using the primers tuf-pGFP-F (SEQ ID NO: 12) and tuf-pGFP-R (SEQ ID NO: 13), the parameters of the PCR amplification being as follows: 95°C 10 min, 95°C 30 s, 65-55°C 30 s, 72°C 3 min, repeating 10 times, 95°C 30 s, 55°C 30 s, 72°C 3 min, repeating 25 times, 72°C with 10 min extension. The above fragments were recovered and then recombinantly ligated via the Vazyme Clon Express Multies Cloning Kit, and the ligated products were transfected into Trans T1 competent cells, coated on a chloramphenicol resistance plate and cultured overnight; positive clones were picked for colony PCR verification; the correct transformants were subjected to sequence confirmation; the obtained recombinant vector was named pXM-P*_{tuf}*-*gfp*. The recombinant vector was transfected into *Corynebacterium glutamicum* ATCC 13032, to obtain recombinant strains. By a method similar to Example 3, intensities of the NCgl1418 promoter and the P*_{tuf}* promoter were compared under the conditions of high salt or normal culture media. The results are shown in Fig. 4; the data shows that under the normal osmolality condition, the NCgl1418 promoter had a lower intensity than P*_{tuf}*, but had substantially the same transcriptional activities with P*_{tuf}* under the high osmolality (adding 0.6M sodium sulfate) condition, indicating that the promoter has a higher intensity under high salt or osmolality conditions and can be used for efficient induced expression of target genes.

### Example 7. Activities of the NCgl1418 promoter at different lengths

With the above constructed pXM-P*_{NCgl1418}*-*gfp* as a template, NCgl1418 promoter fragments of 203 bp (SEQ ID NO: 2), 145 bp (SEQ ID NO: 3), and 94 bp (SEQ ID NO: 4) were respectively obtained through PCR amplification using the primer pairs 1418-203-F/R (SEQ ID NO: 14, 15), 1418-145-F/R (SEQ ID NO: 16, 17), and 1418-94-F/R (SEQ ID NO: 18, 19), the parameters of the PCR amplification being as follows: 95°C 10 min, 95°C 30 s, 65-55°C 30 s, 72°C 3 min, repeating 10 times, 95°C 30 s, 55°C 30 s, 72°C 3 min, repeating 25 times, 72°C with 10 min extension. The three fragments were recovered and phosphorylated using T4 PNK; new vectors were obtained through self-ligation construction and named pXM-P*₂₀₃*-*gfp*, pXM-P*₁₄₅*-*gfp*, and pXM-P*₉₄*-*gfp*, respectively.

The above recombinant pXM-P*₂₀₃*-*gfp*, pXM-P*₁₄₅*-*gfp*, and pXM-P*₉₄*-*gfp* vectors were respectively transfected into *Corynebacterium glutamicum* ATCC 13032, to obtain recombinant strains. By a method similar to Example 3, intensities of the NCgl1418 promoter at different lengths were compared under the conditions of high salt (adding 0.6 M sodium sulfate) and normal culture media. The result is shown in Fig. 5; the data shows that although the NCgl1418 promoter of 94 bp comprises the core sequences (the -35 region and the -10 region), it substantially lost the normal function of the promoter; although the promoter of 145 bp had slightly reduced induction intensity under the high salt condition, it still achieved 74% or more of the activities of the promoter of 243 bp; the promoter of 203 bp substantially kept the activities of the promoter of 243 bp under the high-salt osmolality condition, achieving 94% of the activities of the promoter of 243 bp. The above result indicates that the promoter activities of the NCgl1418 promoter and the activities under the high-salt osmolality condition require comprising at least a length of 145 bp of the DNA sequence as shown in SEQ ID NO: 3.

### Example 8. Application of the NCg11418 promoter regulating dCpf1 in lysine synthesis

According to the genomic sequence (NC _003450.3) of *Corynebacterium glutamicum* ATCC 13032 published by NCBI, the primers 1418-D-F (SEQ ID NO: 20) and 1418-D-R (SEQ ID NO: 21) were designed. With the ATCC 13032 genome as a template, a promoter sequence of the NCgl1418 gene was obtained through PCR amplification, the parameters of the PCR amplification being as follows: 95°C 10 min, 95°C 30 s, 65-55°C 30 s, 72°C 1 min, repeating 10 times, 95°C 30 s, 55°C 30 s, 72°C 1 min, repeating 25 times, 72°C with 10 min extension. Meanwhile, with the pXM-07 reported in the literature as a template^{[11]}, first, the vector fragment 1 having dCpf1 was obtained through PCR amplification using the primers pXM07-F1 (SEQ ID NO: 22) and pXM07-R1 (SEQ ID NO: 23), the parameters of the PCR amplification being as follows: 95°C 10 min, 95°C 30s, 65-55°C 30 s, 72°C 3 min, repeating 10 times, 95°C 30 s, 55°C 30 s, 72°C 3 min, repeating 25 times, 72°C with 10 min extension. Then, the vector fragment 2 comprising the origin of replication was obtained through PCR amplification using the primers pXM07-F2 (SEQ ID NO: 24) and pXM07-R2 (SEQ ID NO: 25), the parameters of the PCR amplification being as follows: 95°C 10 min, 95°C 30 s, 65-55°C 30 s, 72°C 3 min, repeating 10 times, 95°C 30 s, 55°C 30 s, 72°C 3 min, repeating 25 times, 72°C with 10 min extension. With the pEC-26 reported in the literature as a template^{[11]}, the crRNA array fragment targeting the *gltA*, *pgi*, *hom*, and *pck* genes was obtained through PCR amplification using the primers pEC26-F (SEQ ID NO: 26) and pEC26-R (SEQ ID NO: 27), the parameters of the PCR amplification being as follows: 95°C 10 min, 95°C 30 s, 65-55°C 30 s, 72°C 1 min, repeating 10 times, 95°C 30 s, 55°C 30 s, 72°C 1 min, repeating 25 times, 72°C with 10 min extension. The above three fragments were recovered and recombinantly ligated via Vazyme Clon Express Multies One Step Cloning Kit. The ligated products were transfected into Trans T1 competent cells, coated on a chloramphenicol resistance plate and cultured overnight, positive clones were picked for colony PCR verification, the correct transformants were subjected to sequence confirmation, and the obtained recombinant vector was named pXM-P*_{NCgl1418}*-dCpf1. Meanwhile, using pXM-07 as a template, the vector fragment 3 was obtained through PCR amplification using the primers pXM07-F3 (SEQ ID NO: 28) and pXM07-R3 (SEQ ID NO: 29), the parameters of the PCR amplification being as follows: 95°C 10 min, 95°C 30 s, 65-55°C 30 s, 72°C 3 min, repeating 10 times, 95°C 30 s, 55°C 30 s, 72°C 3 min, repeating 30 times, 72°C with 10 min extension. The above fragments were recovered and then recombinantly ligated with the obtained promoter sequence fragments of the NCgl1418 gene via Vazyme Clon Express Multies One Step Cloning Kit. The ligated products were transfected into Trans T1 competent cells, coated on a chloramphenicol resistance plate and cultured overnight, positive clones were picked for colony PCR verification, the correct transformants were subjected to sequence confirmation, to obtain the control pXM-dCpfl-con vector.

According to the lysine strain construction method disclosed in the literature^{[12]}, using the technology of homologous recombination for pK18mobsacB, the threonine at position 311 of aspartate kinase (encoded by lysC) of the *Corynebacterium glutamicum* ATCC13032 genome was mutated to isoleucine, to construct the SCgL30 strain having certain lysine-synthesis capability. The above recombinant pXM-P*_{NCgl1418}*-dCpf1 and pXM-dCpfl-con vectors were respectively transfected into the SCgL30 strain, to obtain recombinant strains and control strains. The above strains were respectively inoculated into TSB culture media containing 5 µg/mL chloramphenicol, cultured overnight at 30°C, 220 r/min, and with the initial OD 0.3, respectively transferred to lysine fermentation culture media to which 0.6 M sodium sulfate was added or not added (simulating the high salt high osmolality environment due to high-concentration product accumulation in the later phase of fermentation). The culture system was a 24-well plate containing 1 mL/well of culture media. The fermentation was terminated after culturing for 24h at 30°C, 800 r/min; and residue glucose, OD₆₀₀, and lysine yield were measured. The ingredients of the lysine fermentation culture media were as follows: glucose, 80 g/L; yeast powder, 8 g/L; urea, 9 g/L; K₂HPO₄, 1.5 g/L; MOPS, 42 g/L; FeSO₄, 0.01 g/L; MnSO₄, 0.01 g/L; MgSO₄, 0.6 g/L; the final chloramphenicol concentration being 5 µg/mL. The measurement result is shown in Table 2; the data shows that when sodium sulfate was not added, the lysine yield and the glucose conversion rate of the strain with attenuated target gene increased by 23% and 25% respectively, as compared with the control strain, indicating that the NCgl1418 promoter was capable of attenuating the target gene by regulating the expression of the target dCpf1 gene. Whilst under the high salt condition of adding 0.6 M sodium sulfate, the lysine yield and the glucose conversion rate of the strain with attenuated target gene increased by 49% and 40%, respectively, as compared with the control strain, indicating a higher expression intensity of dCpf1 in a high salt environment, thereby enhancing the attenuating degree of the target gene and further facilitating the synthesis of lysine and the utilization of the substrate.

**Table 2 Application effect of NCgl1418 promoter regulating dCpf1 expression in lysine synthesis**

| Strain | -Na₂S O₄ | | +Na₂ SO₄ | |
|---|---|---|---|---|
| | lysine (g/L) | glucose conversion rate (g/g) | lysine (g/L) | glucose conversion rate (g/g) |
| SCgL30/pXM-dCpf1-con | 1.95 | 0.033 | 2.35 | 0.049 |
| SCgL30/pXM*-*P*_{NCgl1418}*-dCpf1 | 2.40 | 0.041 | 3.50 | 0.072 |

### Example 9. Application of the NCg11418 promoter regulating lysine transport protein in lysine synthesis

According to the genomic sequence (NC_003450.3) of Corynebacterium glutamicum ATCC 13032 published by NCBI, the primers 1418-L-F (SEQ ID NO: 30) and 1418-L-R (SEQ ID NO: 31), lysE-F (SEQ ID NO: 32) and lysE-R (SEQ ID NO: 33) were designed. With the ATCC 13032 genome as a template, the promoter sequence of the NCgl1418 gene and the DNA sequence of the *lysE* gene were obtained through PCR amplification, respectively, the parameters of the PCR amplification being as follows: 95°C 5 min, 95°C 30 s, 65-55°C 30 s, 72°C 1 min, repeating 10 times, 95°C 30 s, 55°C 30 s, 72°C 1 min, repeating 25 times, 72°C with 10 min extension. Meanwhile, with the pEC-XK99E reported in the literature as a template^{[13]}, vector fragments were obtained through PCR amplification using the primers pEC-F (SEQ ID NO: 34) and pEC-R (SEQ ID NO: 35), the parameters of the PCR amplification being as follows: 95°C 10 min, 95°C 30 s, 65-55°C 30 s, 72°C 3 min, repeating 10 times, 95°C 30 s, 55°C 30 s, 72°C 3 min, repeating 25 times, 72°C with 10 min extension. The above three fragments were recovered and recombinantly ligated via Vazyme Clon Express Multies One Step Cloning Kit. The ligated products were transfected into Trans T1 competent cells, coated on a Kanamycin resistance plate and cultured overnight; positive clones were picked for colony PCR verification; the correct transformants were subjected to sequence confirmation; the obtained recombinant vector was named pEC-P*_{NCgl1418}*-*lysE.*

The above recombinant pEC-P*_{NCgl1418}-lysE* and pEC-XK99E vectors were respectively transfected into *Corynebacterium glutamicum* ScgL30, to obtain recombinant strains and control strains. By, for example, the method of Example 8 (substituting kanamycin of a final concentration 25µg/mL for the antibiotic), the application effect of the strain expressing LysE which is regulated by the NCgl1418 promoter in lysine synthesis was verified, and the result is shown in Fig. 3. The data shows that when sodium sulfate was not added, the lysine yield and the glucose conversion rate of the strain expressing LysE which is regulated by the NCgl1418 promoter were 2.35 g/L and 0.039 g/g, increased by 38% and 40%, respectively, as compared with the control strain; whilst under the high salt condition of adding 0.6 M sodium sulfate, the lysine yield and the glucose conversion rate of the strain expressing LysE reached 3.05 g/L and 0.063 g/g, increased by 49% and 59%, respectively, as compared with the control strain, indicating a higher LysE expression intensity in a high salt environment, thereby facilitating exocytosis and extracellular accumulation of lysine.

**Table 3 Application effect of NCgl1418 promoter regulating LysE expression in lysine synthesis**

| | Strain | -Na₂SO₄ | | +Na₂SO₄ | |
|---|---|---|---|---|---|
| | | lysine (g/L) | glucose conversion rate (g/g) | lysine (g/L) | glucose conversion rate (g/g) |
| SCgL30/pEC-XK99E | | 1.70 | 0.028 | 2.05 | 0.040 |
| SCgL30/pEC-P_{NCgl1418}-*lysE* | | 2.35 | 0.039 | 3.05 | 0.063 |

The above two strategies were further combined, to obtain a recombinant strain in which the NCgl1418 promoter regulates both LysE expression and dCpf1 expression. By, for example, the method of Example 8 (substituting chloramphenicol of a final concentration 5 µg/mL and kanamycin of a final concentration 25 µg/mL for the antibiotic), the application effect of the above recombinant strain in lysine synthesis was verified, and the result is shown in Table 4. The data shows that when sodium sulfate was not added, the strain co-expressing the target genes regulated by the NCgl1418 promoter has a lysine yield increased from 1.75 g/L to 3.15 g/L (increased by 80%) and a glucose conversion rate increased from 0.03 g/g to 0.053 g/g (increased by 78%); whilst under the high salt condition of adding 0.6 M sodium sulfate, the lysine yield and the glucose conversion rate of the strain co-expressing the target genes reached 4.20 g/L and 0.1 g/g, increased by 115% and 127%, respectively, as compared with the control strain, achieving very significant effect.

**Table 4 Application effect of the NCgl1418 promoter simultaneously regulating dCpf1 and LysE expression in lysine synthesis**

| Strain | -Na₂SO₄ | | +Na₂SO₄ | |
|---|---|---|---|---|
| | lysine (g/L) | glucose conversion rate (g/g) | lysine (g/L) | glucose conversion rate (g/g) |
| SCgL30/pEC-XK99E/pXM-dCpf1-con | 1.75 | 0.030 | 1.95 | 0.044 |
| SCgL30/pEC-P*_{NCgl1418}*-*lysE*/pXM-P*_{NCgl1418}*-dCpf1 | 3.15 | 0.053 | 4.20 | 0.100 |

### Example 10. Construction of a library of promoter mutants of the NCgl1418 gene

Since the sequences before and after the -35 region of the promoter may have important controlling effect on the intensity of the promoter, in this example, random mutations were introduced into the sequences before and after the -35 region of the promoter core region (SEQ ID NO: 36) of the *NCgl1418* gene. The sequence of the promoter core region of the *NCgl1418* gene was:
TATTAAAGATCACACCGAGTGGTGGAATTTCCTCAAGTGATTTACCCACAATGG ACTTTG, the underlined were respectively the main sequences of the -35 region and the -10 region of the promoter;

The specific mutant sequence was:

TATTAAAGATCNNNNNNNNNNGTGGAATTTCCTCAAGTGATTTACCCACAATG
GACTTTG.

The primers 1418mutant-F (SEQ ID NO: 49) and 1418mutant-R (SEQ ID NO: 50) were used to perform reverse PCR amplification on pXM-P*_{NCgl1418}*-*gfp* obtained in Example 2; the obtained linearized plasmid fragments were phosphorylated and ligated, and then transfected into *Escherichia coli* T1 competent cells, to obtain resistance clones. All of the obtained strain clones were subjected to cell harvesting and plasmid extraction, to obtain a library of two promoter mutants of the *NCgl1418* gene.

### Example 11. Screening of the library of the promoter mutants of the NCg11418 gene and characterization of the promoter mutants

The library of the promoter mutants was transfected into *Corynebacterium glutamicum* ATCC13032. With the ATCC13032 (pXM-Con) strain and the ATCC13032 (pXM-P*_{NCgl1418}*-*gfp*) strain of Example 3 as an empty vector and a wild-type control, the library of mutants were subjected to positive screening and negative screening for three times. First, the strains were inoculated into TSB culture media containing 5 µg/mL chloramphenicol, cultured for 8 to 10 h at 30°C, 220 r/min, and with the initial OD 1, respectively transferred to CGXIIY culture media to which 0.6 M Na₂SO₄ was added. The culture system was a 24-well plate containing 1 mL/well of culture media. After cultured for 6 h at 30°C, 800 r/min, the obtained bacterial liquid was diluted 1/50 with a PBS buffer solution, subjected to ultrasonic treatment for 6 min, and subjected to fluorescence activated cell sorting by a flow cytometer (positive screening, top 0.01%). The mixed strains obtained through sorting were inoculated into TSB culture media containing 5 µg/mL chloramphenicol, cultured overnight at 30°C, 220 r/min, to be used in the next screening. Then, the mixed strains, the empty vector, and the wild-type control strains were inoculated into TSB culture media containing 5 µg/mL chloramphenicol, cultured for 8 to 10 h at 30°C, 220 r/min, and with the initial OD 0.5, respectively transferred to CGXIIY media; the culture system was a 24-well plate containing 1 mL/well of culture media. After culturing for 6 h at 30°C, 800 r/min, the obtained bacterial liquids were diluted 1/50 with a PBS buffer solution, subjected to ultrasonic treatment for 6 min, and subjected to fluorescence activated cell sorting by a flow cytometer (negative screening, bottom 1%). At last, the process of the first positive screening was repeated.

The strains, the wild-type *NCgl1418* promoter, and the control strain without promoter obtained from the above three times of screening were inoculated into TSB culture media containing 5 µg/mL chloramphenicol, and cultured overnight at 30°C, 220 r/min. The ingredients of the TSB liquid media were as follows (g/L): glucose, 5 g/L; yeast powder, 5 g/L; soy peptone, 9 g/L; urea, 3 g/L; succinic acid, 0.5 g/L; K₂HPO₄•3H₂O, 1 g/L; MgSO₄•7H₂O, 0.1 g/L; biotin, 0.01 mg/L; vitamin B1, 0.1 mg/L; MOPS, 20 g/L.

With the initial OD 0.5, the strains were respectively transferred to CGXIIY culture media to which 0.6 M Na₂SO₄ was added or not added. The culture system was a 24-well plate containing 1 mL/well of culture media. After culturing for 24 h at 30°C, 800 r/min, GFP fluorescence intensity and OD₆₀₀ were measured for different strains. The relative intensity of the promoter mutants was characterized by the fluorescence intensity per strain cell (subtracting the fluorescence intensity per strain cell of the control strain under the same conditions). The ingredients of the CGXIIY culture media were as follows: glucose, 50 g/L; NH₄Cl, 16.5 g/L; urea, 5 g/L; KH₂PO₄, 1 g/L; K₂HPO₄, 1 g/L; MOPS, 42 g/L; MgSO₄, 0.25 g/L; FeSO₄·2H₂O, 0.01 g/L; MnSO₄·H₂O, 0.01 g/L; ZnSO₄·7H₂O, 0.001 g/L; CuSO₄, 0.2 mg/L; NiCl·6H₂O, 0.02 mg/L; CaCl₂, 0.01 g/L; protocatechuic acid, 0.03 g/L; biotin, 0.2 mg/L; Vitamin B1, 0.1 mg/L; the final chloramphenicol concentration being 5 µg/mL. According to the measured fluorescence intensity, 12 strong inducible promoter mutants were obtained, with 1.85 to 7.62 folds of the promoter intensity of the wild-type promoter under the high salt condition, and all keeping an induction activity of 1.64 times or more. The promoter intensity of the 35P7 promoter is 3.9 foldsof the promoter intensity of the wild-type promoter and the induction activity reached 8.46 folds, all significantly higher than that of the wild-type promoter (see Table 5).

**Table 5**

| Promot er | Relativ e intensit y^{a} | Induced activities^{b} | promoter core sequence (mutation region underlined) | Complete promoter sequence (SEQ ID NO) |
|---|---|---|---|---|
| WT | 1.00 | 4.03 | TATTAAAGATCACACCGAGTGGTG GAATTTCCTCAAGTGATTTACCCAC AATGGACTTTG | SEQ ID NO: 1 |
| 35P1 | 2.11 | 2.08 | TATTAAAGATCTACTTGCAGAGTGG AATTTCCTCAAGTGATTTACCCACA ATGGACTTTG | SEQ ID NO: 37 |
| 35P2 | 1.85 | 2.34 | TATTAAAGATCAGTGCTGAAAGTG GAATTTCCTCAAGTGATTTACCCAC AATGGACTTTG | SEQ ID NO: 38 |
| 35P3 | 3.23 | 2.48 | TATTAAAGATCGCACGAAAGGGTG GAATTTCCTCAAGTGATTTACCCAC AATGGACTTTG | SEQ ID NO: 39 |
| 35P4 | 3.07 | 2.02 | TATTAAAGATCTATCTAGAGGGTGG AATTTCCTCAAGTGATTTACCCACA ATGGACTTTG | SEQ ID NO: 40 |
| 35P5 | 3.57 | 2.29 | TATTAAAGATCAGGCTTGTCGGTGG AATTTCCTCAAGTGATTTACCCACA ATGGACTTTG | SEQ ID NO: 41 |
| 35P6 | 2.78 | 2.20 | TATTAAAGATCCGCTTCTTTCGTGG AATTTCCTCAAGTGATTTACCCACA ATGGACTTTG | SEQ ID NO: 42 |
| 35P7 | 3.90 | 8.46 | TATTAAAGATCTAACTCTTGGGTGG AATTTCCTCAAGTGATTTACCCACA ATGGACTTTG | SEQ ID NO: 43 |
| 35P8 | 3.28 | 1.98 | TATTAAAGATCCCAAGTTCCAGTGG AATTTCCTCAAGTGATTTACCCACA ATGGACTTTG | SEQ ID NO: 44 |
| 35P9 | 2.62 | 2.11 | TATTAAAGATCCGGTGCCACAGTG GAATTTCCTCAAGTGATTTACCCAC AATGGACTTTG | SEQ ID NO: 45 |
| 35P10 | 2.70 | 1.95 | TATTAAAGATCAGCAGTTAGGGTG GAATTTCCTCAAGTGATTTACCCAC AATGGACTTTG | SEQ ID NO: 46 |
| 35P11 | 4.47 | 1.64 | TATTAAAGATCAGATAAATAAGTGG AATTTCCTCAAGTGATTTACCCACA ATGGACTTTG | SEQ ID NO: 47 |
| 35P12 | 7.62 | 1.70 | TATTAAAGATCATCGATCTAGGTGG AATTTCCTCAAGTGATTTACCCACA ATGGACTTTG | SEQ ID NO: 48 |

| | | | | |
|---|---|---|---|---|
| ^{a}fluorescence intensity of respective promoters/fluorescence intensity of the wild-type promoter under high salt condition (adding Na₂SO₄) ^{b}fluorescence intensity when Na₂SO₄ was added/fluorescence intensity when Na₂SO₄ was not added | | | | |

### Example 12. Facilitating lysine synthesis by regulating dCpf1 with promoter mutants

According to the genomic sequence (NC_003450.3) of Corynebacterium glutamicum ATCC 13032 published by NCBI, the primers 1418-D-F (SEQ ID NO: 21) and 1418-D-R (SEQ ID NO: 22) were designed; with the ATCC 13032 genome as a template, promoter sequences of the *NCgl1418* gene were obtained through PCR amplification. Meanwhile, with the pXM-07 reported in the literature as a template^{[2]}, first, the vector fragment 1 was obtained through PCR amplification using the primers pXM07-F1 (SEQ ID NO: 23) and pXM07-R1 (SEQ ID NO: 24). Then, the vector fragment 2 comprising the origin of replication was obtained through PCR amplification using the primers pXM07-F2 (SEQ ID NO: 25) and pGFP-R (SEQ ID NO: 20). With the pEC-26 reported in the literature as a template^{[2]}, crRNA array fragments targeting the *gltA*, *pgi*, *hom*, and *pck* genes were obtained through PCR amplification using the primers pEC26-F (SEQ ID NO: 26) and pEC26-R (SEQ ID NO: 27). The above three fragments were recovered and then recombinantly ligated via Vazyme Clon Express Multies One Step Cloning Kit, to obtain the recombinant pXM-P*_{NCgl1418}*-dCpf1 vector. Meanwhile, using pXM-07 as a template, the vector fragment 3 was obtained through PCR amplification using the primers pXM07-F1 (SEQ ID NO: 23) and pGFP-R (SEQ ID NO: 20). The above fragments were recovered and then recombinantly ligated with the obtained promoter sequence fragments of the *NCgl1418* gene via Vazyme Clon Express Multies One Step Cloning Kit, to obtain the control pXM-dCpfl-con vector.

Using pXM-P*_{NCgl1418}*-dCpf1 as a template, two vector fragments (comprising the 35P7 promoter) were obtained through PCR amplification using the primers 35P7-F (SEQ ID NO: 51) and pXM-R (SEQ ID NO: 55), pXM-F (SEQ ID NO: 54) and 35P7-R1 (SEQ ID NO: 52), respectively, and recombinantly ligated to each other via Vazyme Clon Express Multies One Step Cloning Kit, to obtain the recombinant pXM-P_{35P7}-dCpf1 vector. Likewise, using pXM-dCpfl-con as a template, the corresponding control pXM-dCpf1-P_{35P7}con vector was constructed using the same primers and by the same method.

According to the lysine strain construction method disclosed in the literature^{[3]}, using the technology of homologous recombination for pK18mobsacB, the threonine at position 311 of aspartate kinase (encoded by lysC) of the *Corynebacterium glutamicum* ATCC13032 genome was mutated to isoleucine, to construct the SCgL30 strain having certain lysine-synthesis capability. The above recombinant pXM-P_{35P7}-dCpf1, pXM-P*_{NCgl1418}*-dCpf1, pXM-dCpf1-P_{35P7}con, and pXM-dCpfl-con vectors were respectively transfected into the SCgL30 strain, to obtain recombinant strains and control strains. The above strains were inoculated into TSB culture media containing 5 µg/mL chloramphenicol, cultured overnight at 30°C, 220 r/min, and with the initial OD 0.3, respectively transferred to lysine fermentation culture media to which 0.6 M sodium sulfate was added or not added (simulating a high salt high osmolality environment due to high-concentration product accumulation in the later phase of fermentation). The culture system was a 24-well plate containing 1 mL/well of culture media. After culturing for 26 h at 30°C, 800 r/min, the fermentation was terminated, and the glucose residue, OD₆₀₀, and the lysine yield were measured. The ingredients of the lysine fermentation culture media were as follows: glucose, 80 g/L; yeast powder, 8 g/L; urea, 9 g/L; K₂HPO₄, 1.5 g/L; MOPS, 42 g/L; FeSO₄, 0.01 g/L; MnSO₄, 0.01 g/L; MgSO₄, 0.6 g/L; the final chloramphenicol concentration being 5 µg/mL. The measurement result is shown in Table 6 and Table 7; the data shows that when sodium sulfate was not added, the lysine yield and the glucose conversion rate of the strain with attenuated target gene increased by 28% and 31%, respectively, as compared with the control strain, higher than the wild-type *NCgl1418* promoter (increased by 23% and 25%); whilst under the high salt condition of adding 0.6 M sodium sulfate, the lysine yield and the glucose conversion rate of the strain with attenuated target gene increased by 52% and 55%, respectively, as compared with the control strain, significantly higher than the wild-type *NCgl1418* promoter (increased by 49% and 40%). The above result indicates a higher expression intensity of dCpf1 in a high salt environment, thereby increasing the weakened degree of the target gene, further facilitating synthesis of lysine and utilization of the substrate.

**Table 6 Application effect of the 35P7 promoter regulating dCpf1 expression in lysine synthesis**

| Strain | -Na₂SO₄ | | +Na₂SO₄ | |
|---|---|---|---|---|
| | lysine (g/L) | glucose conversion rate (g/g) | lysine (g/L) | glucose conversion rate (g/g) |
| SCgL30/pXM-dCpf1-P_{35P7}con | 2.13 | 0.035 | 2.87 | 0.058 |
| SCgL30/pXM-P_{35P7}-dCpf1 | 2.73 | 0.045 | 4.37 | 0.089 |

**Table 7 Application effect of the wild-type promoter of NCgl1418 regulating dCpf1 expression in lysine synthesis**

| Strain | -Na₂SO₄ | | +Na₂SO₄ | |
|---|---|---|---|---|
| | lysine (g/L) | glucose conversion rate (g/g) | lysine (g/L) | glucose conversion rate (g/g) |
| SCgL30/pXM-dCpf1-con | 1.95 | 0.033 | 2.35 | 0.049 |
| SCgL30/pXM-P*_{NCgl1418}*-dCpf1 | 2.40 | 0.041 | 3.50 | 0.072 |

### Example 13. Application of promoter mutants regulating LysE expression in lysine synthesis

According to the genomic sequence (NC _003450.3) of *Corynebacterium glutamicum* ATCC 13032 published by NCBI, the primers 1418-L-F (SEQ ID NO: 28) and 1418-L-R (SEQ ID NO: 29), lysE-F (SEQ ID NO: 30) and lysE-R (SEQ ID NO: 31) were designed respectively. With the ATCC 13032 genome as a template, the promoter sequence of the *NCgl1418* gene and the DNA sequence of the *lysE* gene were obtained through PCR amplification. Meanwhile, with the pEC-XK99E reported in the literature as a template^{[4]}, vector fragments were obtained through PCR amplification using the primers pEC-F (SEQ ID NO: 32) and pEC-R (SEQ ID NO: 33). The above three fragments were recovered and recombinantly ligated via Vazyme Clon Express Multies One Step Cloning Kit. The ligated products were transfected into Trans T1 competent cells, coated on a Kanamycin resistance plate and cultured overnight, positive clones were picked for colony PCR verification, the correct transformants were subjected to sequence confirmation, the obtained recombinant vector was named pEC-P*_{NCgl1418}*-*lysE.* Using pEC-P*_{NCgl1418}-lysE* as a template, vector fragments comprising the 35P7 promoter mutant and having the *lysE* gene were obtained through PCR amplification using the primers 35P7-F (SEQ ID NO: 51) and 35P7-R2 (SEQ ID NO: 53), and then phosphorylated using T4 PNK; and pEC-P_{35P7}-*lysE* was obtained through self-ligation construction.

The above recombinant pEC-P*_{NCgl1418}*-*lysE*, pEC-P_{35P7}-*lysE*, and pEC-XK99E vectors were respectively transfected into *Corynebacterium glutamicum* ScgL30, to obtain recombinant strains and control strains. By, for example, the method of Example 12 (substituting kanamycin of a final concentration 25µg/mL for the antibiotic), the application effect of the strains expressing LysE regulated by the NCgl1418 promoter in lysine synthesis was verified, and the result is shown in Table 8 and Table 9. The data shows that when sodium sulfate was not added, the lysine yield and the glucose conversion rate of the strain expressing LysE regulated by the 35P7 promoter were 3.00 g/L and 0.050 g/g, increased by 45% and 52%, respectively, as compared with the control strain, and achieved an increase higher than the increase of 38% and 40% achieved by the wild-type *NCgl1418* promoter; whilst under the high salt condition of adding 0.6 M sodium sulfate, the lysine yield and the glucose conversion rate of the strain expressing LysE reached 5.53 g/L and 0.128 g/g, increased by 124% and 176%, respectively, as compared with the control strain, higher than the increase of 49% and 59% achieved by the wild-type *NCgl1418* promoter, exhibiting a good application effect.

**Table 8 Application result of the 35P7 promoter regulating LysE expression in lysine synthesis**

| | strain | | -Na₂SO₄ | | | +Na₂SO₄ | |
|---|---|---|---|---|---|---|---|
| | | lysine (g/L) | | glucose conversion rate (g/g) | lysine (g/L) | | glucose conversion rate (g/g) |
| SCgL30/pEC-XK99E | | 2.07 | | 0.033 | 2.47 | | 0.046 |
| SCgL30/pEC-P_{35P7}-*lysE* | | 3.00 | | 0.050 | 5.53 | | 0.128 |

**Table 9 Application effect of the wild-type promoter of NCgl1418 regulating LysE expression in lysine synthesis**

| | strain | | -Na₂SO₄ | | | +Na₂SO₄ | |
|---|---|---|---|---|---|---|---|
| | | lysine (g/L) | | glucose conversion rate (g/g) | lysine (g/L) | | glucose conversion rate (g/g) |
| SCgL30/pEC-XK99E | | 1.70 | | 0.028 | 2.05 | | 0.040 |
| SCgL30/pEC-P_{NCgl1418}-*lysE* | | 2.35 | | 0.039 | 3.05 | | 0.063 |

In addition, Fig. 5 of the present disclosure proves that the promoter of 145 bp achieved 74% or more of the activity of the promoter of 243 bp under the high salt and osmolality condition; the promoter of 203 bp substantially maintained the activity of the promoter of 243 bp under the high salt and osmolality condition, achieving 94% of the activity of the promoter of 243 bp. It means that since the promoter fragments of SEQ ID NO: 2 and SEQ ID NO: 3 comprise the promoter core region of the *NCgl1418* gene, the promoter fragments of SEQ ID NO: 2 and SEQ ID NO: 3 can also exhibit obviously enhanced promoter activities in an environment of increased salt concentration and osmolality. Therefore, by the method of the examples for reconstructing the promoter core region, i.e., by mutating one or more positions among positions 130 to 139 of the sequence as shown in SEQ ID NO: 2 or mutating one or more positions among positions 72 to 81 of the sequence as shown in SEQ ID NO: 3, a promoter mutant of the same enhanced induction activity can be obtained.

### Example 14. Construction of plasmids having endogenous promoter sequence of the proP gene

The above obtained Ncgl1418 promoter mutant was obtained by mutating a partial sequence of the core region of the wild-type Ncgl1418 promoter (TATTAAAGATCACACCGAGTGGTGGAATTTCCTCAAGTGATTTACCCACAATGGACT TTG, the underlined being the main sequences of the -35 region and the -10 region of the promoter) and exhibited obviously enhanced promoter activity as compared with the wild-type promoter in an environment of increased salt concentration and osmolality; that is, the induction activity of Ncgl1418 can be further enhanced by modification. Therefore, the present disclosure intends to further improve the induction activity of the promoter by substitution of the whole sequence of the complete core region sequence, the 5'-UTR sequence, and the sequence of other regions of the promoter.

Similar to the Ncgl1418 promoter, which exhibits enhanced promoter activity in environments with increased salt concentration and osmolality, it has been reported in the literature that the expression of ProP is up-regulated under high osmolality conditions, indicating that the promoter of this gene could be a high osmolality-inducible promoter (Franzel, B., et al., Adaptation of Corynebacterium glutamicum to salt-stress conditions, Proteomics, 2010, 10(3): 445-457.). ProP is a protein that intakes proline and has an induced expression under high osmolality conditions. By increasing the intake of the biocompatible proline, it improves the tolerance of the strains for a high osmolality environment. According to the genomic sequence (NC_003450.3) of *Corynebacterium glutamicum* ATCC 13032 published by NCBI, the primers proP-F (SEQ ID NO: 59) and proP-R (SEQ ID NO: 60) were designed. With the ATCC 13032 genome as a template, a promoter (P*_{proP}*) sequence (SEQ ID NO: 56) having the *prop* gene was obtained through PCR amplification. Meanwhile, with the pXM-*gfp* reported in the literature (Sun DH et al., Journal of Industrial Microbiology & Biotechnology, 2019, 46(2): 203-208.) as a template, vector fragments from which the *lacI* gene and the promoter *tac* were removed were obtained through PCR amplification using the primers pGFP-F (SEQ ID NO: 8) and pGFP-R (SEQ ID NO: 9). The above fragments were recovered and recombinantly ligated via the Vazyme Clon Express Multies Cloning Kit. The ligated products were transfected into Trans T1 competent cells, coated on a chloramphenicol resistance plate and cultured overnight. Positive clones were picked for colony PCR verification. The correct transformants were subjected to sequence confirmation. The obtained recombinant vector was named pXM-P*_{proP}*-*gfp*. Meanwhile, the vector fragments were phosphorylated using T4 PNK; the control pXM-con vector was obtained through self-ligation construction. The above recombinant vector was transfected into the *Corynebacterium glutamicum* ATCC 13032, to obtain a recombinant strain.

### Example 15. Inducing effect of high salt on the promoter of the proP gene

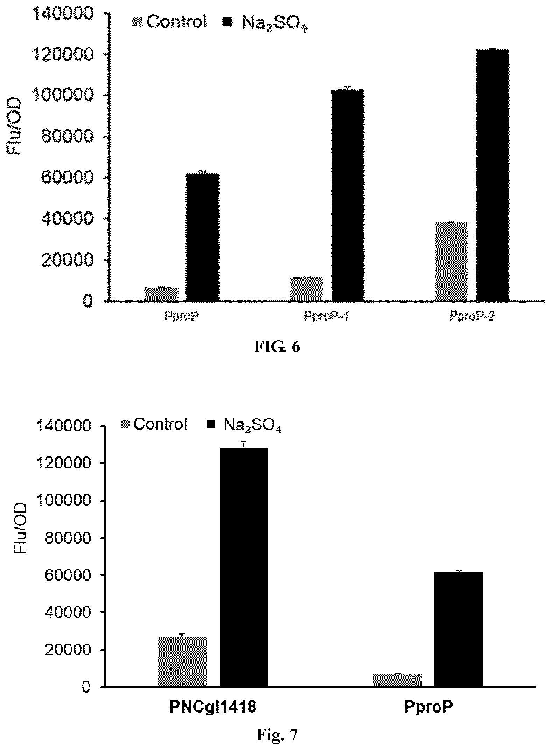

The above strains having recombinant vectors of different promoters were respectively inoculated into TSB culture media containing 5 µg/mL chloramphenicol, cultured overnight at 30°C, 220 r/min. The ingredients of the TSB liquid medium were as follows (g/L): glucose, 5 g/L; yeast powder, 5 g/L; soy peptone, 9 g/L; urea, 3 g/L; succinic acid, 0.5 g/L; K₂HPO₄•3H₂O, 1 g/L; MgSO₄•7H₂O, 0.1 g/L; biotin, 0.01 mg/L; vitamin B1, 0.1 mg/L; MOPS, 20 g/L. With the initial OD 0.5, the strains were transferred to CGXIIY culture media to which 0.6 M Na₂SO₄ was added or not added. The culture system was a 24-well plate containing 1 mL/well of culture media. After culturing for 18 h at 30°C, 800 r/min, GFP fluorescence intensity and OD₆₀₀ were measured for the different strains. The relative intensities of different promoters under different conditions were characterized by the fluorescence intensity per strain cell (subtracting the fluorescence intensity per strain cell of the control strains under the same conditions). The ingredients of the CGXIIY culture medium were: glucose, 50 g/L; yeast powder, 2 g/L; NH₄Cl, 16.5 g/L; urea, 5 g/L; KH₂PO₄, 1 g/L; K₂HPO₄, 1 g/L; MOPS, 42 g/L; MgSO₄, 0.25 g/L; FeSO₄·2H₂O, 0.01 g/L; MnSO₄·H₂O, 0.01 g/L; ZnSO₄·7H₂O, 0.001 g/L; CuSO₄, 0.2 mg/L; NiCl·6H₂O, 0.02 mg/L; CaCl₂, 0.01 g/L; protocatechuic acid, 0.03 g/L; biotin, 0.2 mg/L; Vitamin B1, 0.1 mg/L; the final chloramphenicol concentration being 5 µg/mL. The result is shown in Fig. 6; the data shows that the promoter of the *prop* gene was induced by high salt and achieved 8.9 times induction activity. But, as compared with the activity of the NCgl1418 promoter as measured in Example 7, the promoter of the *proP* gene had obviously lower intensity than the NCgl1418 promoter (see Fig. 7).

### Example 16. Hybrid reconstruction of the Ncgl1418 promoter and the proP promoter and construction of characterization plasmids

In view of the high induction activity of the *proP* promoter and the high expression intensity of the *NCgl1418* promoter, in order to obtain a promoter having both a high induction activity and a high expression intensity, the present disclosure further verified the effect of the hybrid reconstruction of the *Ncgl1418* promoter and the *proP* promoter. First, the possible regulatory region of the *proP* promoter was remained to maintain the high induction activity; then the 5'-UTR of the *proP* promoter was substituted with the 5'-UTR of the *Ncgl1418* promoter (SEQ ID NO: 57), or the -35 region and the -10 region of the core region as well as the 5'-UTR of the *proP* promoter were substituted with the -35 region and the -10 region of the core region as well as the 5'-UTR of the *Ncgl1418* promoter (SEQ ID NO: 58), to maintain the high expression intensity of the promoter. As such, two promoter mutants P*_{proP-1}* (SEQ ID NO: 57) and P*_{proP-2}* (SEQ ID NO: 58) were obtained.

Using pXM-P*_{proP}*-*gfp* as a template, the 5'-UTR region following the *proP* promoter was substituted through PCR amplification using the primers proP-1-F (SEQ ID NO: 61) and proP-1-R (SEQ ID NO: 62). The above PCR fragments were recovered and phosphorylated using T4 PNK; pXM-P*_{proP-1}*-*gfp* was obtained through self-ligation construction. Meanwhile, using pXM-P*_{proP}*-*gfp* as a template, the 5'-UTR region following the *proP* promoter and the core region of the -35 region and the -10 region were substituted through PCR amplification using the primers proP-2-F (SEQ ID NO: 63) and proP-2-R (SEQ ID NO: 64). The above fragments were recovered and phosphorylated using T4 PNK; pXM-P*_{proP-2}*-*gfp* was obtained through self-ligation construction. The recombinant vector was transfected into *Corynebacterium glutamicum* ATCC 13032, to obtain a recombinant strain.

### Example 17. Inducing effect of high salt on the hybrid promoter

By the method similar to Example 15, the intensity and the induction activity of the promoter mutants were compared under the conditions of high salt or normal culture medium. The results are shown in Fig. 7; the data shows that the two modified promoters both had higher intensity than the wild-type *proP* promoter under the conditions of normal culture medium and high salt. The induction activity of P*_{proP-1}* substantially maintained the performance of the wild-type *proP* promoter (8.7 times); the induction activity of P*ₚᵣₒₚ₋₂* reduced to 3.2 times due to its relatively high base expression level.

### Example 18. Facilitating lysine synthesis by using the promoter mutants to regulate dCpf1 expression

Using pXM-P*_{proP}-gfp* as a template, the primers proP-D-F (SEQ ID NO: 65) and proP-D-R (SEQ ID NO: 66) were designed, fragments of the P*_{proP}* promoter were obtained through PCR amplification. Meanwhile, with the pXM-07 reported in the literature as a template^{[5]}, first, the vector fragment 1 having dCpf1 was obtained through PCR amplification using the primers pXM07-F1 (SEQ ID NO: 22) and pXM07-R1 (SEQ ID NO: 23). Then, the vector fragment 2 comprising the origin of replication was obtained through PCR amplification using the primers pXM07-F2 (SEQ ID NO: 24) and pXM07-R2 (SEQ ID NO: 25). With the pEC-26 reported in the literature (Li MY et al., Frontiers in Bioengineering and Biotechnology, 2020, 8: 357.) as a template, the crRNA array fragment targeting the *gltA*, *pgi*, *hom*, and *pck* genes were obtained through PCR amplification using the primers pEC26-F (SEQ ID NO: 26) and pEC26-R (SEQ ID NO: 27). The above four fragments were recovered and recombinantly ligated via Vazyme Clon Express Multies One Step Cloning Kit, to obtain the recombinant pXM-P*_{proP}*-dCpf1 vector. Likewise, using pXM-P*_{proP-1}-gfp* as a template, the primers proP-D-F (SEQ ID NO: 65) and proP1-D-R (SEQ ID NO: 67) were designed, and fragments of the P_{*proP*-*1*} promoter were obtained through PCR amplification. The promoter fragment and the vector fragment 1, the vector fragment 2, and the crRNA array fragment were recombined to obtain the recombinant pXM-P*_{proP-1}*-dCpf1 vector. Meanwhile, using pXM-P*_{proP}*-dCpf1 as a template, the vector fragment 3 was obtained through PCR amplification using the primers pXM07-F1 (SEQ ID NO: 22) and pXM07-R2 (SEQ ID NO: 25). The above fragments were recovered and recombinantly ligated with the fragments of the P*_{proP}* and P*_{proP-1}* promoter sequences via Vazyme Clon Express Multies One Step Cloning Kit, to obtain the corresponding control pXM-P*_{proP}*-con and pXM-P*_{proP-1}*-con vectors.

According to the lysine strain construction method disclosed in the literature (Becker, J., et al., Metab. Eng., 2011, 13, 159-168.), the threonine at position 311 of aspartate kinase (encoded by lysC) of the Corynebacterium glutamicum ATCC13032 genome was mutated to isoleucine using the technology of homologous recombination for pK18mobsacB, to construct the SCgL30 strain having certain lysine-synthesis capability. The recombinant pXM-P*_{proP}*-dCpf1 and pXM-P*_{proP-1}*-dCpf1 vectors, and their respective control pXM-P*_{proP}*-con and pXM-P*_{proP-1}*-con plasmids were respectively transfected into the SCgL30 strain, to obtain recombinant strains and control strains. The above strains were respectively inoculated into TSB culture media containing 5 µg/mL chloramphenicol, cultured overnight at 30°C, 220 r/min, and with the initial OD 0.3, respectively transferred to lysine fermentation culture media to which 0.6 M sodium sulfate was added or not added (simulating the high salt high osmolality environment due to high-concentration product accumulation in the later phase of fermentation). The culture system was a 24-well plate containing 1 mL/well of culture media. After culturing for 24h at 30°C, 800 r/min, the fermentation was terminated, and glucose residue, OD₆₀₀, and lysine yield were measured. The ingredients of the lysine fermentation culture media were as follows: glucose, 80 g/L; yeast powder, 8 g/L; urea, 9 g/L; K₂HPO₄, 1.5 g/L; MOPS, 42 g/L; FeSO₄, 0.01 g/L; MnSO₄, 0.01 g/L; MgSO₄, 0.6 g/L; the final chloramphenicol concentration being 5 µg/mL. The measurement result is shown in Table 10. The result shows that due to the relatively weak promoter intensity, the dCpf1 attenuating system regulated the wild-type *proP* promoter had very limited effect on the lysine yield and the conversion rate; and the reconstructed promoter mutants did not have obvious effects under normal culturing conditions. But under the high osmolality inducement conditions, the dCpf1 system regulated by the promoter mutants exhibited good application effects, with the lysine yield and the conversion rate being significantly increased.

**Table 10 Application effect of the hybrid promoter regulating dCpf1 expression in lysine synthesis**

| strain | -Na₂SO₄ | | +Na₂SO₄ | |
|---|---|---|---|---|
| | lysine (g/L) | glucose conversion rate (mM/M) | lysine (g/L) | glucose conversion rate (mM/M) |
| SCgL30/pXM-P*_{proP}*-con | 2.1 | 40.8 | 1.9 | 37.1 |
| SCgL30/pXM-P*_{proP}*-dCpf1 | 2.2 | 43.0 | 2.2 | 43.6 |
| SCgL30/pXM-P*_{proP-1}*-con | 2.1 | 41.0 | 1.9 | 37.7 |
| SCgL30/pXM-P*_{proP-1}-*dCpf1 | 2.3 | 49.0 | 2.9 | 60.6 |

### Example 19. Application of the promoter mutants regulating LysE expression in lysine synthesis

Using pXM-P*_{proP}*-*gfp* as a template, fragments of the P*_{proP-1}* promoter sequence were obtained through PCR amplification using the primers proP-lysE-F (SEQ ID NO: 68) and proP-lysE-R (SEQ ID NO: 69); using pXM-P*_{proP-1}-gfp* as a template, fragments of the P*_{proP}* promoter sequence were obtained through PCR amplification using the primers proP-lysE-F (SEQ ID NO: 68) and proP1-lysE-R (SEQ ID NO: 70). With the ATCC 13032 genome as a template, fragments of the *lysE* gene were obtained through PCR amplification using the primers lysE-F (SEQ ID NO: 32) and lysE-R (SEQ ID NO: 33). Meanwhile, using pXM-XK99E as a template, vector fragments were obtained through PCR amplification using the primers pEC-F (SEQ ID NO: 34) and pEC-R (SEQ ID NO: 35). The above promoter fragments were recombinantly ligated with the fragments of the *lysE* gene and the vector fragments. The ligated products were transfected into Trans T1 competent cells, coated on a Kanamycin resistance plate and cultured overnight, positive clones were picked for colony PCR verification, the correct transformants were subjected to sequence confirmation, the obtained recombinant vectors were named pEC-*P_{proP}-lysE* and pEC-P*_{proP-1}-lysE.*

The above recombinant pEC-P*_{proP}-lysE*, *pEC-P_{proP-1}-lysE*, and pEC-XK99E vectors were respectively transfected into *Corynebacterium glutamicum* ScgL30, to obtain recombinant strains and control strains. By, for example, the method of Example 17 (substituting kanamycin of a final concentration 25µg/mL for the antibiotic), the application effects of different strains expressing LysE regulated by different promoters in lysine synthesis were verified, and the result is shown in Table 11. The data shows that the wild-type *prop* promoter regulating LysE overexpression slightly increased the lysine yield and the conversion rate under the high osmolality condition, whilst the modified hybrid promoter achieves significant increase of the lysine yield and the conversion rate under the high osmolality inducement condition, exhibiting a good application effect.

**Table 11 Application effect of different promoters regulating LysE expression in lysine synthesis**

| strain | | -Na₂SO₄ | | | +Na₂SO₄ | |
|---|---|---|---|---|---|---|
| | lysine (g/L) | | glucose conversion rate (mM/M) | lysine (g/L) | | glucose conversion rate (mM/M) |
| SCgL30/pEC-XK99E | 2.1 | | 40.7 | 2.1 | | 40.8 |
| SCgL30/pEC-P*_{proP}*-*lysE* | 2.1 | | 40.7 | 2.3 | | 46.1 |
| SCgL30/pEC-P*_{proP-1}*-*lysE* | 2.3 | | 44.0 | 2.9 | | 65.8 |

### Reference documents:

[10] Sun DH et al., Journal of Industrial Microbiology & Biotechnology 2019, 46(2): 203-208.
[11] Li MY et al., Frontiers in Bioengineering and Biotechnology, 2020, 8: 357.
[12] Becker, J., et al., Metab. Eng., 2011, 13, 159-168.
[13] O Kirchner, et al. Journal of Biotechnology, 2003, 104: 287-299.

All technical features disclosed in this specification can be combined in any way of combination. Each feature disclosed in this specification can also be replaced by another feature having the same, equal or similar function. Therefore, unless otherwise specified, each feature disclosed herein is merely an example of a series of equal or similar features.

In addition, according to the above description of the present disclosure, those skilled in the art can easily understand the key features of the present disclosure, and many modifications can be made to the invention to adapt to various use purposes and conditions without departing from the spirit and scope of the present disclosure, and therefore such modifications are also intended to fall within the scope of the appended claims.

## Claims

1. A polynucleotide having promoter activity, wherein the polynucleotide is selected from any one of a group consisting of (i) to (viii):
(i) comprising a nucleotide sequence as shown in any one sequence of SEQ ID NOs: 1 to 3;
(ii) a mutant of the polynucleotide having the sequence as shown in SEQ ID NO: 1, the mutant having a mutated nucleotide at one or more positions corresponding to positions 170 to 179 of the sequence as shown in SEQ ID NO: 1; the activity of the mutant is higher than the promoter activity of the polynucleotide having the sequence as shown in SEQ ID NO: 1, and the nucleotide sequence of the mutant at positions corresponding to positions 170 to 179 in the sequence as shown in SEQ ID NO: 1 is not ACACCGAGTG;
(iii) a mutant of the polynucleotide having the sequence as shown in SEQ ID NO: 2, the mutant having a mutated nucleotide at one or more positions corresponding to positions 130 to 139 in the sequence as shown in SEQ ID NO: 2; the activity of the mutant is higher than the promoter activity of the polynucleotide having the sequence as shown in SEQ ID NO: 2, and a nucleotide sequence of the mutant at positions corresponding to positions 130 to 139 in the sequence as shown in SEQ ID NO: 2 is not ACACCGAGTG;
(iv) a mutant of a polynucleotide having the sequence as shown in SEQ ID NO: 3, the mutant having a mutated nucleotide at one or more positions corresponding to positions 72 to 81 in the sequence as shown in SEQ ID NO: 3; the mutant having an activity higher than the promoter activity of the polynucleotide having the sequence as shown in SEQ ID NO: 3, and a nucleotide sequence of the mutant at positions corresponding to positions 72 to 81 in the sequence as shown in SEQ ID NO: 3 is not ACACCGAGTG;
(v) comprising a nucleotide sequence having the sequence as shown in any one of SEQ ID NOs: 57 to 58;
(vi) a polynucleotide having a reverse complementary sequence to the nucleotide sequence as set forth in any one of (i) to (v);
(vii) a polynucleotide having a reverse complementary sequence to a sequence t capable of hybridizing with the nucleotide sequence as set forth in any one of (i) to (v) under high-stringent hybridization conditions or very high-stringent hybridization conditions;
(viii) a polynucleotide having a sequence of having at least 80%, optionally at least 90%, preferably at least 95%, more preferably at least 97%, further preferably at least 98%, most preferably at least 99% sequence identity to the nucleotide sequence as shown in any one of (i) to (v).

2. The polynucleotide having promoter activity according to claim 1, wherein the polynucleotide has an enhanced promoter activity in an environment of increased salt concentration or osmolality.

3. The polynucleotide having promoter activity according to claim 1 or 2, wherein the nucleotide sequence of the mutant at positions corresponding to positions 170 to 179 in the sequence as shown in SEQ ID NO: 2, or corresponding to positions 130 to 139 in the sequence as shown in SEQ ID NO: 3, or corresponding to positions 72 to 81 in the sequence as shown in SEQ ID NO: 4, is any one selected from a group consisting of (p₁) to (p₁₂):
(p₁) TACTTGCAGA,
(p₂) AGTGCTGAAA,
(p₃) GCACGAAAGG,
(p₄) TATCTAGAGG,
(p₅) AGGCTTGTCG,
(p₆) CGCTTCTTTC,
(p₇) TAACTCTTGG,
(p₈) CCAAGTTCCA,
(p₉) CGGTGCCACA,
(p₁₀) AGCAGTTAGG,
(p₁₁) AGATAAATAA,
(p₁₂) ATCGATCTAG.

4. The polynucleotide having promoter activity according to any one of claims 1 to 3, wherein a nucleotide sequence of the mutant is selected from a sequence as shown in any one of SEQ ID NOs: 37 to 48.

5. A transcription expression cassette, wherein the transcription expression cassette comprises the polynucleotide having promoter activity according to any one of claims 1 to 4; optionally, the transcription expression cassette further comprises a protein-coding gene, the protein-coding gene is operably ligated with the polynucleotide having promoter activity.

6. A recombinant expression vector, wherein the recombinant expression vector comprises the polynucleotide having promoter activity according to any one of claims 1 to 4, or the transcription expression cassette according to claim 5.

7. A recombinant host cell, wherein the recombinant host cell comprises the transcription expression cassette according to claim 5, or the recombinant expression vector according to claim 6.

8. The recombinant host cell according to claim 7, wherein the host cell is from genus *Corynebacterium*, genus *Brevibacterium*, genus *Arthrobacterium*, genus *Microbacterium*, or genus *Escherichia*; preferably, the host cell is *Corynebacterium glutamicum* or *Escherichia coli*; more preferably, the host cell is *Corynebacterium glutamicum* ATCC 13032, *Corynebacterium glutamicum* ATCC 13869, or *Corynebacterium glutamicum* ATCC 14067 and derivative strains thereof.

9. Use of the polynucleotide having promoter activity according to any one of claims 1 to 4, the transcription expression cassette according to claim 5, the recombinant expression vector according to claim 6, the recombinant host cell according to claim 7 or 8 in at least one of
(a) regulation of the transcriptional level of a gene, or preparation of a reagent or kit for regulating a transcriptional level of a gene;
(b) preparation of a protein, or preparation of a reagent or kit for preparing a protein;
(c) production of target compounds, or preparation of a reagent or kit for producing target compounds.

10. The use according to claim 9, wherein the protein is selected from a gene expression regulatory protein or a protein involved in the synthesis of a target compound, a gene expression regulatory protein or a protein involved in membrane transport.

11. The use according to claim 9 or 10, wherein the target compound includes at least one of an amino acid and an organic acid; optionally, the amino acid is one of or a combination of two or more of proline, hydroxyproline, lysine, glutamic acid, threonine, glycine, alanine, valine, leucine, isoleucine, serine, cysteine, glutamine, methionine, aspartic acid, asparagine, arginine, histidine, phenylalanine, tyrosine, tryptophan, 5-aminolevulinic acid or derivatives of any one of the above amino acids; optionally, the organic acid is one of or a combination of two or more of: citric acid, succinic acid, lactic acid, acetic acid, butyric acid, palmitic acid, oxalic acid, oxaloacetic acid, tartaric acid, propionic acid, hexenoic acid, capric acid, caprylic acid, valeric acid, malic acid, or a derivative of the above organic acids.

12. A method for regulating the transcription of a target gene, wherein the method comprises a step of operably ligating the polynucleotide having promoter activity according to any one of claims 1 to 4 with a target gene; or the method comprises a step of operably ligating the polynucleotide having promoter activity according to any one of claims 1 to 4 with a target RNA;
optionally, the target gene comprises at least one of a coding gene encoding a protein involved in the synthesis of a target compound, a coding gene encoding a gene expression regulatory protein, or a coding gene encoding a protein involved in membrane transport;
optionally, the target RNA is at least one of tRNA, sRNA.

13. A method for preparing a protein, comprising a step of expressing the protein using the transcription expression cassette according to claim 5, the recombinant expression vector according to claim 6, or the recombinant host cell according to claim 7 or 8; optionally, the protein is a protein involved in the synthesis of a target compound, a protein involved in membrane transport, or a gene expression regulatory protein;
optionally, the method further comprises a step of isolating or purifying the protein.

14. A method for producing a target compound, comprising a step of expressing a protein involved in the synthesis of a target compound, a protein involved in membrane transport, or a gene expression regulatory protein using the transcription expression cassette according to claim 5, the recombinant expression vector according to claim 6, or the recombinant host cell according to claim 7 or 8, and producing the target compound in an environment where the protein involved in the synthesis of the target compound, a protein involved in membrane transport, or the gene expression regulatory protein is present;
optionally, the target compound includes at least one of an amino acid or an organic acid; optionally, the amino acid is one of or a combination of two or more of: proline, hydroxyproline, lysine, glutamic acid, threonine, glycine, alanine, valine, leucine, isoleucine, serine, cysteine, glutamine, methionine, aspartic acid, asparagine, arginine, histidine, phenylalanine, tyrosine, tryptophan, 5-aminolevulinic acid or derivatives of any one of the above amino acids; optionally, the organic acid is one of or a combination of two or more of citric acid, succinic acid, lactic acid, acetic acid, butyric acid, palmitic acid, oxalic acid, oxaloacetic acid, tartaric acid, propionic acid, hexenoic acid, capric acid, caprylic acid, valeric acid, malic acid, or a derivative or any one of the above organic acids;
optionally, the method further comprises a step of isolating or purifying the target compound.

15. The method according to claim 14, wherein the protein involved in synthesis of target compounds is one of or a combination of two or more of: pyruvate carboxylase, phosphoenolpyruvate carboxylase, γ-glutamyl kinase, glutamate semialdehyde dehydrogenase, pyrroline-5-carboxylate reductase, amino acidtransport protein, ptsG system, pyruvate dehydrogenase, homoserine dehydrogenase, oxaloacetate decarboxylase, glucose-repressible protein, glucose dehydrogenase, aspartate kinase, aspartate semialdehyde dehydrogenase, aspartate ammonia lyase, dihydrodipicolinate synthase, dihydrodipicolinate reductase, dihydropyridinic acid reductase, succinyl diaminopimelate aminotransferase, tetrahydrodipicolinate succinylase, succinyl diaminopimelate deacylase, diaminopimelic acid epimerase, diaminopimelic acid deacylase, glyceraldehyde-3-phosphate dehydrogenase, transketolase, diaminopimelate dehydrogenase;
optionally, the protein involved in the synthesis of the target compound is a protein involved in the synthesis of L-amino acids;
optionally, the protein involved in the synthesis of the target compound is a protein involved in the synthesis of lysine.
